# EUROPEAN PATENT APPLICATION

(11) **EP 4 306 215 A1**
(43) Date of publication of application: **17.01.2024**
(21) Application number: 23178233.5
(22) Date of filing: 08.06.2023
(51) Int. Cl.: B01L 3/00, B01L 3/02, C12M 3/06

(54) **FLUID HANDLING SYSTEM**

(30) Priority: 13.07.2022 US 202217864112
(71) Applicant: Enplas Corporation, Kawaguchi-shi, Saitama 332-0034 (JP)
(72) Inventor: SUZUKI, Seiichiro, Kawaguchi-shi, 332-0034 (JP)
(74) Representative: J A Kemp LLP

(57) **Abstract**

Provided is a novel fluid handling system capable of preventing bubbles from entering an internal flow channel of a fluid handling device during fluid delivery to the internal flow channel. A fluid handling system (1) of the present invention includes a fluid handling device (10), a reservoir (20) that stores a fluid, and a supply unit (31) that supplies the fluid to the reservoir (20), wherein the fluid handling device(10) has a first flow channel (101), the reservoir (20) has a main body (203), the main body (203) having a communicating portion (202) that communicates with the first flow channel (101) of the fluid handling device (10), and the supply unit (31) has a second flow channel through which the fluid flows and a vent portion (314) that makes the second flow channel communicate with the outside, the second flow channel having an inlet port (312) through which the fluid flows into the second flow channel and an outlet port (313) through which the fluid flows out of the second flow channel, the vent portion (314) being located above the outlet port (313), the outlet port (313) being located inside the reservoir (20), and the vent portion (314) being located above the communicating portion (202).

## Description

### TECHNICAL FIELD

The present invention relates to a fluid handling system.

### BACKGROUND ART

In recent years, organs-on-chips that reproduce biological functions through the formation of blood vessel models in micro-flow channels of the chips have been attracting attention in the fields of cancer immunology and the like from the viewpoint of, for example, reducing animal experiments and improving research efficiency. As a specific example, it is possible to construct a blood vessel on an inner wall of a micro-flow channel of an organ-on-a-chip by delivering a vascular cell fluid and the like to the micro-flow channel, and furthermore, it is possible to reproduce the behavior of a target solvent such as a drug solution in a blood vessel of a living body by delivering the target solvent to the micro-flow channel and circulating it therethrough (WO 2016/174724).

Fluid delivery to the organ-on-a-chip is performed by an automated system using, for example, a fluid delivery device such as a pump. However, if bubbles are mixed in the fluid, the bubbles may flow into the micro-flow channel as well. In the case of a micro-flow channel in which a blood vessel model or the like is formed, there is a risk that bubbles entering the micro-flow channel will cause, for example, blood cells adhering to the inner wall of the micro-flow channel to peel off, or blood cells adhering to each other to be dissociated.

WO 2016/174724 is an example of related art.

### SUMMARY OF THE INVENTION

Therefore, it is an object of the present invention is to provide a novel fluid handling system capable of preventing bubbles entering a flow channel of a fluid handling device during fluid delivery to the flow channel, for example.

To achieve the above-described object, a fluid handling system of the present invention includes a fluid handling device, a reservoir that stores a fluid, and a supply unit that supplies the fluid to the reservoir,
wherein the fluid handling device has a first flow channel,
the reservoir has a main body,
   the main body having a communicating portion that communicates with the first flow channel of the fluid handling device, and
the supply unit has a second flow channel through which the fluid flows and a vent portion that makes the second flow channel communicate with an outside,
   the second flow channel having an inlet port through which the fluid flows into the second flow channel and an outlet port through which the fluid flows out of the second flow channel,
   the vent portion being located above the outlet port,
   the outlet port being located inside the reservoir, and
   the vent portion being located above the communicating portion.

A supply unit for a fluid handling system of the present invention is a fluid supply unit for the above-described fluid handling system of the present invention, the supply unit including a flow channel through which a fluid flows and a vent portion that makes the flow channel communicate with an outside,
wherein the flow channel has an inlet port through which the fluid flows into the flow channel and an outlet port through which the fluid flows out of the flow channel, and
the vent portion is located above the communicating portion.

A kit for a fluid handling system of the present invention is a kit unit for the above-described fluid handling system of the present invention, the kit including a reservoir that stores a fluid and a supply unit that supplies the fluid to the reservoir,
wherein the reservoir has a main body,
the main body having a communicating portion that communicates with the first flow channel of the fluid handling device, and
the supply unit has a flow channel through which the fluid flows and a vent portion that makes the flow channel communicate with an outside,
   the flow channel having an inlet port through which the fluid flows into the flow channel and an outlet port through which the fluid flows out of the flow channel,
   the vent portion being located above the outlet port,
   the outlet port being located inside the reservoir, and
   the vent portion being located above the communicating portion.

A method for supplying a fluid of the present invention uses the above-described fluid handling system of the present invention including the fluid handling device, the reservoir that stores a fluid, and the supply unit that supplies the fluid to the reservoir, and includes:
introducing a fluid into the reservoir via the supply unit; and
introducing the fluid stored in the reservoir into the first flow channel of the fluid handling device from the reservoir.

In the fluid handling system of the present invention, since the supply unit has the vent portion, even when bubbles enter the supply unit along with the fluid, the bubbles are preferentially released through the vent portion to the outside, rather than through the outlet port through which the fluid flows out. Therefore, when the fluid flows out of the supply unit, bubbles can be prevented from accumulating near the outlet port, through which the fluid flows out of the supply unit. Consequently, for example, the entry of bubbles into the flow channel of the fluid handling device as described above can be prevented.

### BRIEF DESCRIPTION OF THE DRAWINGS

Fig. 1 is a schematic diagram showing an example of a fluid handling system of Embodiment 1.
Figs. 2A to 2D are schematic diagrams showing an example of a supply unit of Embodiment 1; Fig. 2Ais a perspective view, Fig. 2B is a side view, Fig. 2C is a cross-sectional view taken in direction I-I, and Fig. 2D is a cross-sectional view taken in direction II-II.
Fig. 3 is a schematic diagram showing the relationship between the supply unit and a reservoir of Embodiment 1.
Figs. 4A and 4B are schematic diagrams showing other supply unit examples of Embodiment 2; Fig. 4A is a side view of a supply unit having a rectangular vent portion, and Fig. 4B is a side view of a supply unit having an elliptical vent portion.
Fig. 5 is a schematic diagram showing the relationship between a supply unit and a reservoir of Embodiment 3.
Fig. 6 is a schematic diagram showing changes in fluid level of a fluid layer in the reservoir of Embodiment 3.
Fig. 7 is a schematic diagram showing the relationship between a supply unit and a reservoir of Embodiment 4.
Fig. 8 is a schematic diagram showing the state of bubbles released from a supply unit of Example 1.
Fig. 9 is a schematic diagram showing the state of bubbles released from a supply unit of Comparative Example 1.
Figs. 10A and 10B are schematic diagrams, each showing the state of bubbles released from a supply unit of Example 2; Fig. 10A is the schematic diagram with respect to a supply unit having a rectangular vent portion, and Fig. 10B is the schematic diagram with respect to a supply unit having a circular vent portion.
Figs. 11A and 11B are schematic diagrams showing an example of a fluid handling system of Embodiment 5; Fig. 11A is a top view, and Fig. 11B is a bottom view.
Figs. 12A and 12B are schematic diagrams showing the example of the fluid handling system of Embodiment 5; Fig. 12Ais a side view of the fluid handling system shown in Figs. 11A and 11B, and Fig. 12B is a cross-sectional view taken in direction IX-IX in Fig. 11A.
Figs. 13A to 13E are schematic diagrams showing the relationship between a supply unit and a reservoir of Embodiment 5; Fig. 13Ais a top view, Fig. 13B is a perspective view, Fig. 13C is a side view, Fig. 13D is a cross-sectional view taken in direction X-X in Fig. 11A (direction II-II in Fig. 13A), and Fig. 13E is a cross-sectional view of the supply unit taken in direction III-III in Fig. 13C.
Figs. 14 is a schematic diagram showing a reservoir with a supply unit in the fluid handling system of Embodiment 5.
Fig. 15 is a schematic diagram showing the state of bubbles released from the supply unit of Embodiment 5.
Fig. 16 is a schematic diagram showing an example of a fluid handling system.
Fig. 17 is a schematic diagram showing an example of a reservoir of a fluid handling system.

### DETAILED DESCRIPTION OF THE INVENTION

[1] A fluid handling system including a fluid handling device, a reservoir that stores a fluid, and a supply unit that supplies the fluid to the reservoir,
   wherein the fluid handling device has a first flow channel,
   the reservoir has a main body,
      the main body having a communicating portion that communicates with the first flow channel of the fluid handling device, and
   the supply unit has a second flow channel through which the fluid flows and a vent portion that makes the second flow channel communicate with an outside,
      the second flow channel having an inlet port through which the fluid flows into the second flow channel and an outlet port through which the fluid flows out of the second flow channel,
      the vent portion being located above the outlet port,
      the outlet port being located inside the reservoir, and
      the vent portion being located above the communicating portion.
[2] The fluid handling system according to clause [1], wherein the vent portion is a slit or a through hole.
[3] The fluid handling system according to clause [2], wherein the supply unit has the inlet port located above the outlet port, and the vent portion between the outlet port and the inlet port.
[4] The fluid handling system according to clause [2] or [3], wherein an end portion of the vent portion on the inlet port side is located above a fluid level position to which the fluid is designed to be stored in the reservoir.
[5] The fluid handling system according to clause [1], wherein the outlet port of the supply unit is a slit or a through hole.
[6] The fluid handling system according to clause [5], wherein the supply unit has the outlet port located above the inlet port, and the vent portion located above the inlet port and the outlet port.
[7] The fluid handling system according to clause [5] or [6], wherein an end portion of the outlet port of the supply unit on the inlet port side is located below a fluid level position to which the fluid is designed to be stored in the reservoir.
[8] The fluid handling system according to any one of clauses [1] to [7],
   wherein a ratio of a cross-sectional area (R) of the main body of the reservoir to a cross-sectional area (T) of the supply unit is 1.5 or more, and
   the cross-sectional area (T) and the cross-sectional area (R) are each the area of a horizontal cross section passing through an end portion of the vent portion on the inlet port side.
[9] The fluid handling system according to any one of clauses [1] to [8], wherein the main body of the reservoir has a fluid storage volume of 0.05 to 10 mL.
[10] The fluid handling system according to any one of clauses [1] to [9], wherein the main body of the reservoir is open at its top.
[11] The fluid handling system according to any one of clauses [1] to [10],
   wherein the reservoir has the main body and a lid portion,
   the main body is open at its top, and
   the lid portion is a lid portion that covers a top opening of the main body, and has an insertion hole for the supply unit.
[12] The fluid handling system according to any one of clauses [1] to [11], further including a circulation flow channel,
   wherein the fluid handling device has an inlet port through which the fluid from the reservoir flows into the fluid handling device and an outlet port through which the fluid flows out of the fluid handling device, and
   the outlet port of the fluid handling device and the inlet port of the supply unit are connected to each other by the circulation flow channel.
[13] The fluid handling system according to clause [12], wherein the circulation flow channel is a tube.
[14] The fluid handling system according to clause [12] or [13],
   wherein the circulation flow channel is a tube, and
   the circulation flow channel and the supply unit are integrated.
[15] The fluid handling system according to any one of clauses [1] to [14], further including a fluid delivery unit.
[16] A supply unit for a fluid handling system, which is a fluid supply unit for the fluid handling system according to any one of clauses [1] to [15], the supply unit including a flow channel through which a fluid flows and a vent portion that makes the flow channel communicate with an outside,
   wherein the flow channel has an inlet port through which the fluid flows into the flow channel and an outlet port through which the fluid flows out of the flow channel, and
   the vent portion is located above the outlet port.
[17] The supply unit for the fluid handling system according to clause [16], wherein the vent portion is a slit or a through hole.
[18] A kit for the fluid handling system according to any one of clauses [1] to [15], the kit including a reservoir that stores a fluid and a supply unit that supplies the fluid to the reservoir,
   wherein the reservoir has a main body,
   the main body having a communicating portion that communicates with the first flow channel of the fluid handling device, and
   the supply unit has a flow channel through which the fluid flows and a vent portion that makes the flow channel communicate with an outside,
      the flow channel having an inlet port through which the fluid flows into the flow channel and an outlet port through which the fluid flows out of the flow channel,
      the vent portion being located above the outlet port,
      the outlet port being located inside the reservoir, and
      the vent portion being located above the communicating portion.
[19] The kit for the fluid handling system according to clause [18], wherein the vent portion is a slit or a through hole.
[20] The kit for the fluid handling system according to clause [18] or [19], wherein an end portion of the vent portion on the inlet port side is located above a fluid level position to which the fluid is designed to be stored in the reservoir.
[21] The kit for the fluid handling system according to any one of clauses [18] to [20],
   wherein a ratio of a cross-sectional area (R) of the main body of the reservoir to a cross-sectional area (T) of the supply unit is 1.5 or more, and
   the cross-sectional area (T) and the cross-sectional area (R) are each the area of a horizontal cross section passing through an end portion of the vent portion on the inlet port side.
[22] The kit for the fluid handling system according to any one of clauses [18] to [21], wherein the main body of the reservoir has a fluid storage volume of 0.05 to 10 mL.
[23] The kit for the fluid handling system according to any one of clauses [18] to [22],
   wherein the reservoir has the main body and a lid portion,
   the main body is open at its top, and
   the lid portion is a lid portion that covers a top opening of the main body, and has an insertion hole for the supply unit.
[24] A method for delivering a fluid using the fluid handling system according to any one of clauses [1] to [15] including the fluid handling device, the reservoir that stores a fluid, and the supply unit that supplies the fluid to the reservoir, the method including:
   introducing a fluid into the reservoir via the supply unit; and
   introducing the fluid stored in the reservoir into the first flow channel of the fluid handling device from the reservoir.
[25] The method for delivering a fluid according to clause [24],
   wherein the reservoir has a main body that is open at its top,
   the fluid that is stored in the reservoir and introduced into the first flow channel of the fluid handling device from the reservoir is an aqueous solvent,
   the method further includes introducing a non-affinity solvent that has no affinity for the aqueous solvent into the reservoir to form a non-affinity solvent layer of the non-affinity solvent on an aqueous solvent layer of the aqueous solvent, and
   the formation of the non-affinity solvent layer makes the aqueous solvent layer stored in the reservoir a closed system.
[26] The method for delivering a fluid according to clause [25], wherein the non-affinity solvent is an oily solvent.
[27] The method for delivering a fluid according to clause [24],
   wherein the reservoir has a main body and a lid portion,
   the main body is open at its top, and
   the lid portion is a lid portion that covers a top opening of the main body, and has an insertion hole for the supply unit.

### [Fluid Handling System]

As described above, a fluid handling system of the present invention includes a fluid handling device, a reservoir that stores a fluid, and a supply unit that supplies the fluid to the reservoir,
wherein the fluid handling device has a first flow channel,
the reservoir has a main body, the main body having a communicating portion that communicates with the first flow channel of the fluid handling device, and
the supply unit has a second flow channel through which the fluid flows and a vent portion that makes the second flow channel communicate with the outside, the second flow channel having an inlet port through which the fluid flows into the second flow channel and an outlet port through which the fluid flows out of the second flow channel, the vent portion being located above the outlet port, the outlet port being located inside the reservoir, and the vent portion being located above the communicating portion. In the present invention, the vent portion may be paraphrased as the "vent portion for the second flow channel". The fluid handling system of the present invention may also be referred to as a "fluid delivery system".

In the present invention, the vent portion is located above the outlet port in the supply unit as described above, and, for example, it is preferable that the vent portion is at least partially located above the outlet port. As specific examples, a form in which the vent portion is partially located above the outlet port, or in other words, a form in which the entire vent portion is located extending upward from the outlet port may be adopted, or a form in which the entire vent portion is located above the outlet port, or in other words, a form in which the entire vent portion is located above and away from the outlet port of the supply unit may be adopted.

In the present invention, the vent portion is located above the communicating portion of the reservoir as described above, and, for example, it is preferable that entire vent portion is located above the communicating portion. As specific examples, a form in which the vent portion is located above the communicating portion with no contact therebetween, a form in which the entire vent portion is located above and away from the communicating portion, and the like are preferable.

In the present invention, the term "horizontal" means a direction that is parallel to a plane (horizontal plane) perpendicular to the direction of gravity. In addition, the terms "up", "above", "down", and "below" mean the positional relationships when, for example, the fluid handling system of the present invention is being used.

In the present invention, the flow channel of the fluid handling device and the flow channel of the supply unit are referred to as the first flow channel and the second flow channel, respectively, for the sake of convenience in order to distinguish between the two flow channels, but the terms "first" and "second" here are not intended to limit the present invention in any respect.

Hereinafter, specific examples of the present invention will be described using the drawings. In the drawings, the same portions are denoted by the same reference numerals. The present invention is neither limited nor restricted in any respect by embodiments below. The description of each embodiment can be applied to the other embodiments unless otherwise stated.

### (Embodiment 1)

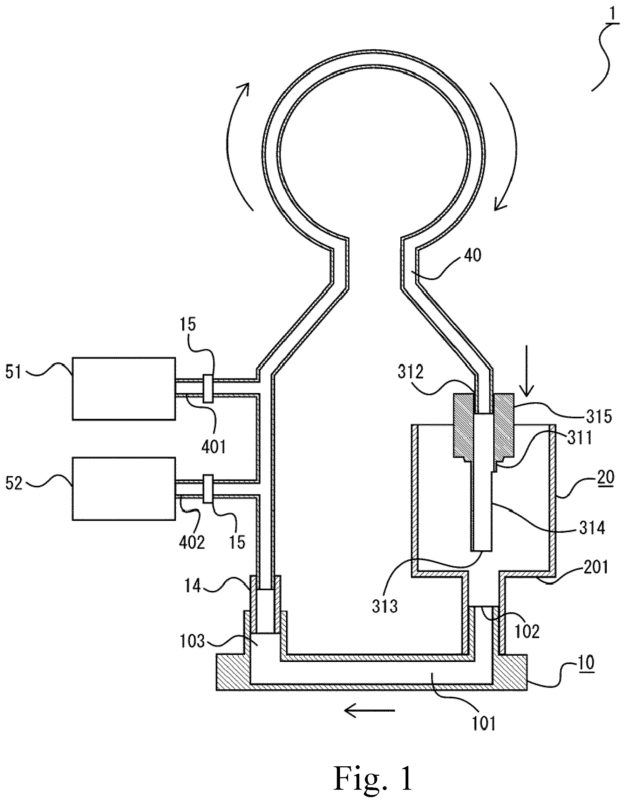

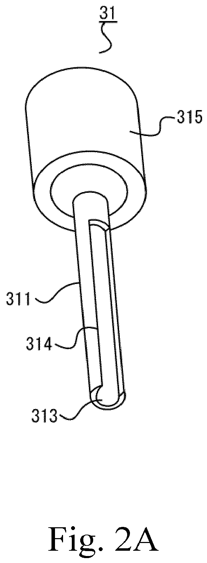

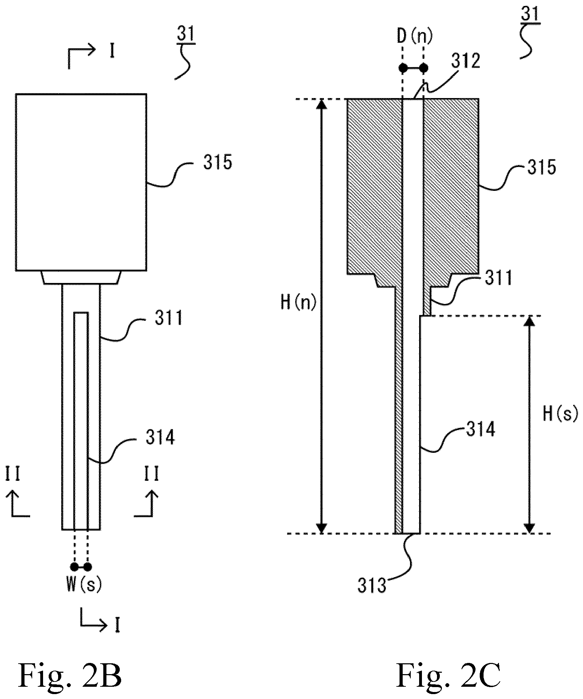

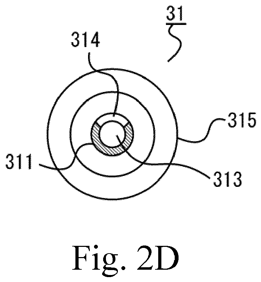

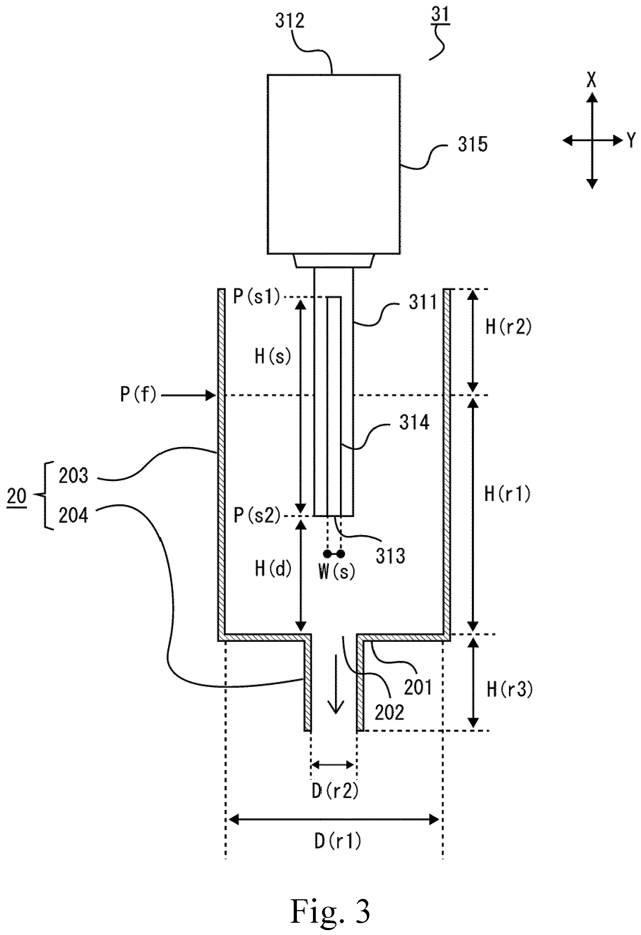

Examples of a fluid handling system of the present embodiment and a partial configuration thereof are shown in the schematic diagrams of Figs. 1 to 3. Fig. 1 is a schematic diagram showing a fluid handling system 1. Fig. 1 shows some of the constituent elements of the fluid handling system 1 in cross section, but this schematic diagram is given to illustrate the connection relationship between the individual constituent elements as well as the flow of a fluid, and the vertical direction and other directions are not limited to those in Fig. 1. Figs. 2A to 2D are schematic diagrams showing an example of a supply unit 31 of the fluid handling system 1. Fig. 2Ais a perspective view of the supply unit 31, Fig. 2B is a side view of the supply unit 31, Fig. 2C is a cross-sectional view of the supply unit 31 taken in direction I-I, and Fig. 2D is a cross-sectional view of the supply unit 31 taken in direction II-II. Fig. 3 is a schematic diagram showing a reservoir 20 and the supply unit 31 extracted from the fluid handling system 1 in Fig. 1. Fig. 3 is a partial cross-sectional view, and shows a cross-sectional view of the reservoir 20 and a side view of the supply unit 31 for the sake of convenience. These figures only schematically show examples of the fluid handling system of the present embodiment and the constituent elements thereof, and the present invention is not limited to the structure, shape, size, method of use, and the like shown in these figures.

In Fig. 3, a direction X is the direction of gravity, and a direction Y is a horizontal direction or a direction that is orthogonal to the direction X. The direction X can also be said to be, for example, the axial direction of the supply unit 31 or the direction of flow of the fluid in the supply unit 31. In Fig. 3, P(f) indicates a fluid level position to which the fluid is designed (planned, desired) to be stored in the reservoir 20. P(f) indicates the fluid level position to which the fluid is designed to be stored, and does not preclude the fluid level of the stored fluid from becoming above or below P(f) during actual use, for example. Hereinafter, P(f) is referred to as the designed fluid level position.

As shown in Fig. 1, the fluid handling system 1 includes a fluid handling device 10, the reservoir 20, and the supply unit 31. The fluid handling device 10 has a first flow channel 101. One end of the first flow channel 101 constitutes an inlet port 102 through which the fluid flows into the first flow channel 101, and the other end of the first flow channel 101 constitutes an outlet port 103 through which the fluid flows out of the first flow channel 101. The first flow channel 101 is a hollow flow channel and is also referred to as the "internal flow channel" of the fluid handling device 10. The reservoir 20 has a main body (also referred to as "main body chamber") 203 that stores the fluid, and the main body 203 has a communicating portion 202 and is in communication with the first flow channel 101 of the fluid handling device 10 via the communicating portion 202. In the following description, the communicating portion 202 of the reservoir 20 is also referred to as the "first flow channel communicating portion". The supply unit 31 has a hollow space (i.e., second flow channel) through which the fluid flows. An opening 312 at an upper end of the second flow channel constitutes an inlet port through which the fluid flows into the second flow channel, and an opening 313 at a lower end of the second flow channel constitutes an outlet port through which the fluid flows out of the second flow channel. In addition, the supply unit 31 has a slit 314 serving as the vent portion for the second flow channel, and the slit 314 is located above the outlet port 313 of the second flow channel. The supply unit 31 is disposed such that the opening (outlet port) 313 at the lower end thereof is located inside the reservoir 20 and the slit (vent portion) 314 is located above the communicating portion 202 of the reservoir 20.

With the fluid handling system 1, as a result of using the supply unit 31 that has the vent portion 314 and disposing the supply unit 31 relative to the reservoir 20 so as to be positioned as described above, bubbles can be prevented from accumulating (staying) near the outlet port 313 of the supply unit 31, and consequently, bubbles can be prevented from entering the first flow channel 101 of the fluid handling device 10.

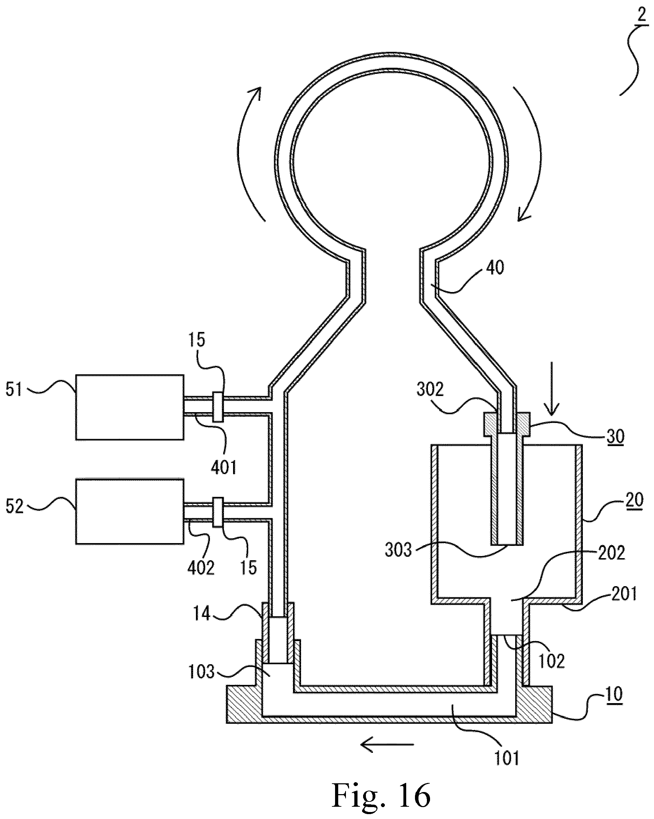

Here, for comparison, an example in which a conventional supply unit that does not have a vent portion is incorporated in a fluid handling system is shown in Fig. 16. Fig. 16 is a schematic diagram showing an example of a fluid handling system 2, and illustrates the same configuration as that of the fluid handling system 1 shown in Fig. 1 of the present embodiment, except that a supply unit 30 that does not have a vent portion is present. However, Fig. 16 only shows the supply unit 30 as a conventional supply unit, and does not show the components other than the supply unit 30 as those of the structure of a conventional fluid handling system. In the case of the fluid handling system 2 incorporating the supply unit 30, since the supply unit 30 does not have a vent portion, if bubbles enter the supply unit 30 through the inlet port 302 of the supply unit 30 along with the fluid, when the fluid flows out through the outlet port 303 of the supply unit 30, the bubbles also flow out and accumulate near the outlet port 303. This causes a problem in that the accumulating bubbles will enter the first flow channel 101 of the fluid handling device 10 via the communicating portion 202 of the reservoir 20.

Furthermore, the accumulating bubbles at the outlet port 303 of the supply unit 30 may combine with bubbles that flow out later to grow to form a large air layer. In particular, the smaller the volume between an outer side surface of the supply unit 30 and an inner side surface of the reservoir 20 in the horizontal direction, the more likely that bubbles accumulating near the outlet port 303 of the supply unit 30 will grow into an air layer. If the air layer is formed in the reservoir 20, the likelihood of bubbles entering the first flow channel 101 of the fluid handling device 10 increases.

In contrast, in the fluid handling system 1 of the present embodiment, as described above, the supply unit 31 has the vent portion 314 above the outlet port 313. Thus, even if bubbles enter the supply unit 31 along with the fluid, the bubbles, which are lighter than the fluid, are released from the supply unit 31 to the outside through the vent portion 314 that is located above the outlet port 313 in the supply unit 31. Therefore, bubbles can be prevented from accumulating near the outlet port 313 of the supply unit 31, and consequently, bubbles can be prevented from entering the first flow channel 101 of the fluid handling device 10. Furthermore, for example, irrespective of the volume between the supply unit 31 and the reservoir 20, the accumulation of bubbles and the growth of bubbles into an air layer can be sufficiently suppressed, and accordingly, the entry of bubbles into the first flow channel 101 of the fluid handling device 10 can be prevented.

Note that, in the fluid handling system of the present invention, for example, the vent portion of the supply unit does not preclude the release of the fluid to the outside therethrough (the same applies to embodiments that will be described later). That is to say, the present invention is characterized in that, as a result of the supply unit having the vent portion, for example, bubbles entering the supply unit can be preferentially released through the vent portion, rather than through the outlet port, and the fluid may also be released through the vent portion.

Now, the fluid handling system 1 of the present embodiment will be described in greater detail. The fluid handling system 1 has a form in which the supply unit 31 is inserted from a top opening of the reservoir 20 toward the inside thereof. In other words, this is a form in which the outlet port 313 side of the supply unit 31 is accommodated into the reservoir 20 from above while pointing downward, and as will be described later, the fluid is supplied from the supply unit 31 into the reservoir 20 located below the supply unit 31.

The supply unit 31 has a form in which the vent portion 314 is partially located above the outlet port 313, or in other words, a form in which the slit-like vent portion 314 is located extending upward from the outlet port 313 of the supply unit 31.

As shown in Figs. 1 to 3, the main body 311 of the supply unit 31 is a hollow tube, and the hollow space (also referred to as "internal space") constitutes the second flow channel. The opening 312 at one end of the tube constitutes the inlet port 312 through which the fluid flows into the inside of the tube, and the opening 313 at the other end of the tube constitutes the outlet port 313 through which the fluid flows out of the inside of the tube. A side surface portion (also referred to as "side wall portion") of the main body 311 of the supply unit 31 has the vent portion 314. The vent portion 314 of the supply unit 31 is also referred to as the "communicating portion for air" that makes the inside and the outside of the second flow channel communicate with each other. The vent portion 314 shown in Figs. 2A to 2D is a slit 314 formed in the side surface portion of the main body 311 of the supply unit 31. In the present embodiment, the slit 314 is a cut formed from the outlet port 313 of the supply unit 31 toward the inlet port 312.

The hollow space (internal space) of the supply unit 31 may have a circular shape or an angular shape in horizontal cross section (cross section in the direction Y), for example. As described above, the direction Y may be, for example, the horizontal direction or a direction that is orthogonal to the flow direction of the fluid. In the following description, a horizontal cross section is a cross section in the horizontal direction and can also be read as a cross section in a direction that is orthogonal to the flow direction. The circular shape may be, for example, a perfect circle or an ellipse, and the angular shape may be, for example, a quadrangle, such as a square or a rectangle, or another polygon. In addition, the horizontal cross section of the hollow space of the supply unit 31 may have, for example, the same shape with a constant size in the direction of gravity or the flow direction of the fluid, or a shape (so-called tapered shape) that becomes smaller in the direction of gravity (downward direction) or the flow direction of the fluid. In the present embodiment, as shown in Figs. 2A to 2D, for example, an inner surface of the supply unit 31 that forms the hollow space has a circular cylindrical shape (preferably a perfect circular cylindrical shape), and the supply unit 31 may have, on an upper side (inlet port 312 side) thereof, a holding portion 315 that protrudes in the horizontal direction (direction Y in Fig. 3).

For example, a tube made of a resin or the like can be used for the supply unit 31, and as specific examples, a plastic tube (elastic tube) made of a soft resin or the like may be used, or a non-plastic tube may be used. The supply unit 31 can also be said to be a nozzle, for example.

Note that, in the present embodiment, an example of the supply unit 31 is described in which the main body 311 is a hollow tube, and the openings at the two ends of the hollow space (the second flow channel) constitute the inlet port 312 and the outlet port 313; however, the present invention is not limited to this. It is sufficient that the main body is, for example, a hollow body having a hollow space that constitutes the second flow channel. The main body is not limited to, for example, the above-described tube (e.g., a circular cylindrical shape), and, in a cross section that is orthogonal to the flow direction of the fluid in the second flow channel, the shape of the second flow channel (hollow space) may be circular, polygonal, or the like, and the external shape of the supply unit may be circular, polygonal, or the like. In addition, in the supply unit 31, the position of the inlet port 312 is not limited to, for example, the opening at one end of the main body 311 and may be an opening formed in a region of the side surface portion on the one end side, for example, and the position of the outlet port 313 is not limited to, for example, the opening at the other end of the main body 311 and may be an opening formed in a region of the side surface portion on the other end side, for example.

As shown in Fig. 3, the reservoir 20 has the main body (also referred to as "main body chamber") 203 that is open at the top. In the reservoir 20, the opening at the top is referred to as the "top opening" in order to distinguish it from the opening 202 that constitutes the first flow channel communicating portion. The first flow channel communicating portion 202 makes the inside of the main body 203 of the reservoir 20 communicate with the inside of the first flow channel 101. The communicating portion 202 is, for example, the opening 202 in a bottom portion 201 of the main body 203, and, for example, the communicating portion 202 may also have a hollow protruding portion 204 that protrudes downward from the opening. The protruding portion 204 can also be said to be a connection portion 204 that is connected to the first flow channel 101 of the fluid handling device 10, for example. In the reservoir 20, the position of the communicating portion 202 is not limited to the bottom portion 201, and may be, for example, a side surface portion of the main body 203, in which case a region of the side surface portion that is close to the bottom portion 201 is preferred. The bottom portion 201 is also referred to as "bottom surface" (the same applies hereinafter).

It is sufficient that the reservoir 20 can store the fluid as described above, and there is no particular limitation on the shape of the reservoir 20. As a specific example, the main body 203 of the reservoir 20 may be, for example, a bottomed cylindrical body. The hollow space (internal space) of the main body 203 may have a circular shape or an angular shape in horizontal cross section (cross section in the direction Y), for example. The circular shape may be, for example, a perfect circle or an ellipse, and the angular shape may be, for example, a quadrangle, such as a square or a rectangle, or another polygon. In addition, the horizontal cross section of the hollow space of the main body 203 may have, for example, the same shape with a constant size in the direction of gravity (downward direction) or the flow direction of the fluid, or a shape that becomes smaller or larger in the direction of gravity (downward direction) or the flow direction of the fluid. The communicating portion 202 in the bottom portion 201 of the reservoir 20 may have, for example, a circular shape or an angular shape. The circular shape may be, for example, a perfect circle, an ellipse, or the like, and the angular shape may be, for example, a quadrangle, such as a square or a rectangle, or another polygon. In the main body 203 of the reservoir 20, as shown in Fig. 3, for example, an inner surface that forms the hollow space has a bottomed circular cylindrical shape (preferably a perfect circular cylindrical shape), and the communicating portion 202 in the bottom portion 201 has a circular shape (preferably a perfect circle). In addition, in the connection portion 204 of the reservoir 20 as well, for example, an inner surface that forms the hollow space has a cylindrical shape, and the hollow space may have a circular shape or an angular shape in horizontal cross section.

As shown in Fig. 3, the lower outlet port 313 side of the supply unit 31 is accommodated in the inside of the reservoir 20 (the inside of the main body 203). At this time, the entire slit-like vent portion 314 of the supply unit 31 is located above and away from the first flow channel communicating portion 202 of the reservoir 20. In Fig. 3, the downward arrowhead indicates the flow direction of the fluid, with the inlet port 312 side of the supply unit 31 on the upper side, and the outlet port 313 side on the lower side. Specifically, for example, the supply unit 31 is accommodated such that its leading end (outlet port 313) on the lower side is located below the designed fluid level position P(f) in the main body 203 of the reservoir 20. In other words, the supply unit 31 is accommodated such that, when the fluid is stored in the reservoir 20, the leading end (outlet port 313) of the supply unit 31 is located in the fluid layer.

Furthermore, it is preferable that the supply unit 31 is disposed such that, for example, an end portion P(s1) of the vent portion 314 on the inlet port 312 side is located above the designed fluid level position P(f) in the reservoir 20. In other words, it is preferable that the supply unit 31 is disposed such that the vent portion 314 of the supply unit 31 is partially located above the fluid level (exposed above the fluid level) in the reservoir 20. This form makes it possible to release bubbles in the supply unit 31 directly into the atmosphere (air layer) above the fluid layer, rather than into the fluid layer stored in the reservoir 20, for example.

According to the present embodiment, the vent portion 314 of the supply unit 31 is a slit that is formed from the outlet port 313 at the lower end toward the inlet port 312, and therefore, for example, even when the fluid level in the reservoir 20 rises or lowers, the vent portion 314 can be used with high versatility. For example, the closer the inlet port 312-side end portion of the vent portion 314 of the supply unit 31 is to the inlet port 312, the more versatile the vent portion 314 becomes with respect to fluctuations in the position of the fluid level of the stored fluid.

The amount of fluid to be stored in the reservoir 20 can be set as appropriate based on, for example, the volume of the internal space of the reservoir 20, the amount of fluid that flows into the reservoir 20 from a fluid chamber 51, which will be described later, and/or the amount of fluid that is discharged to a collection chamber 52, which will be described later. Therefore, the designed fluid level position P(f) in the reservoir 20 can be determined based on, for example, the size of the reservoir 20 used, the fluid delivery amount, and the fluid discharge amount.

In the fluid handling system 1, the inlet port 312 of the supply unit 31 and the outlet port 103 of the first flow channel 101 of the fluid handling device 10 may be connected to each other via a circulation flow channel 40. One end of the circulation flow channel 40 may be, for example, directly connected to the inlet port 312 of the supply unit 31, and the other end of the circulation flow channel 40 may be, for example, directly connected to the outlet port 103 of the fluid handling device 10 or may be connected to the outlet port 103 via a hollow connection portion 14 as shown in Fig. 1.

The fluid handling system 1 may further include, for example, the fluid chamber 51 and the collection chamber 52. The fluid chamber 51 is a chamber in which the fluid to be flowed into the circulation flow channel 40 is stored, and is connected to the circulation flow channel 40 via a branch flow channel 401. An openable valve 15 that controls the fluid flowing out of the fluid chamber 51 is disposed in the branch flow channel 401. The collection chamber 52 is a chamber in which the fluid collected from the circulation flow channel 40 is stored, and is connected to the circulation flow channel 40 via a branch flow channel 402. An openable valve 15 that controls the fluid flowing into the collection chamber 52 is disposed in the branch flow channel 402. In the fluid handling system 1, the fluid is delivered in the flow direction (direction indicated by the arrows in Fig. 1) in the circulation flow channel 40 by driving a fluid delivery unit (not shown).

There is no particular limitation on the type of the circulation flow channel 40, and, for example, a tube may be used. The tube is a hollow member, for example. For example, a tube made of a resin or the like can be used as the tube, and specific examples include a plastic tube (also referred to as "elastic tube") made of a soft resin or the like, and a non-plastic tube. The circulation flow channel 40 is not limited to the tube, and may also be, for example, a flow channel (hereinafter also referred to as "substrate flow channel") that is formed by covering a flow-channel-like recess in a substrate with a cover. The cover may be, for example, a soft cover, or preferably an elastic cover. For example, the description of a contact region 414 of a circulation flow channel 41 of Embodiment 5, which will be described later, can be applied to this form. The circulation flow channel 40 may also be, for example, a combination of the tube and the substrate flow channel.

The fluid delivery unit is a fluid delivery device that delivers the fluid present in the circulation flow channel 40 by, for example, applying a pressure to the circulation flow channel 40. For example, a pressing device having a pressing portion that presses against an outer surface of the circulation flow channel 40 can be used as the fluid delivery unit. There is no particular limitation on the type of the pressing device, and examples include a roller pump (also called a peristaltic pump), a rotary pump (including a rotary membrane pump), and the like. The roller pump includes, for example, a rotating roller as the pressing portion, and can deliver the fluid present in the circulation flow channel 40 as a result of the rotating roller pressing against the outer surface of the circulation flow channel 40. The rotary pump includes, for example, a sliding portion as the pressing portion, and can deliver the fluid present in the circulation flow channel 40 as a result of the sliding portion pressing against the outer surface of the circulation flow channel 40 while sliding thereon. In this case, for example, a contact region (also referred to as "pressed region") of the circulation flow channel 40 that comes into contact with the pressing portion is soft, or preferably elastic. The circulation flow channel 40 is preferably soft, elastic, or the like, for example, and the material thereof is not particularly limited and may be a resin, for example. The type of the circulation flow channel 40 can be selected according to, for example, the type of the above-described pressing device. When the pressing device is the above-described roller pump, for example, the above-described tube is preferably used as the circulation flow channel 40. When the pressing device is the above-described rotary pump, for example, the above-described substrate flow channel is preferably used as the circulation flow channel 40, and a form is also preferable in which the contact region is constituted by the above-described substrate flow channel while the other region is constituted by the above-described tube. In the latter case, for example, an opening at an end of the substrate flow channel and an opening of the tube can be made to communicate with each other.

In addition, the fluid delivery unit may also be, for example, a pressure generator that creates a negative pressure or a positive pressure inside the circulation flow channel 40. The type of the pressure generator is not particularly limited and may be, for example, a pressure pump or the like. The pressure pump may deliver the fluid, for example, by creating a negative pressure inside the circulation flow channel 40 and thereby pulling the fluid, or by creating a positive pressure inside the circulation flow channel 40 and thereby pushing the fluid. In this case, for example, the pressure pump may be connected to the circulation flow channel 40 so that a negative pressure or a positive pressure can be created inside the circulation flow channel 40. Specifically, for example, the inside of the circulation flow channel 40 and the pressure pump are made to communicate with each other.

For example, the supply unit 31 and the circulation flow channel 40 may be separate members that are independent of each other or may be integrated into a single molded body. In the latter case, the circulation flow channel 40 and the supply unit 31 are constituted by, for example, a single integrated tube in which they are in communication with each other.

There is no particular limitation on the fluid that is delivered to the fluid handling system 1, and examples include an aqueous solvent, an organic solvent, and the like. Examples of the aqueous solvent include solvents such as water, a buffer solution, and a medium, and the aqueous solvent may also contain, for example, various components such as growth factors and nutritional components, reagents, and the like according to the purpose. Examples of the organic solvent include alcohols such as ethanol.

Next, with respect to the fluid handling system 1, the sizes of the reservoir 20 and the supply unit 31 will be described by way of example; however, their sizes are not limited to the examples below. Also, the sizes of the other components of the fluid handling system 1 are not particularly limited and can be set as appropriate according to, for example, the sizes of the reservoir 20 and the supply unit 31. Hereinafter, the sizes of the reservoir 20 and the supply unit 31 will be described taking as an example of a case in which both the inside of the reservoir 20 and the inside of the supply unit 31, which form the hollow spaces, have a circular cylindrical shape; however, the present invention is not limited to this. Furthermore, in the case where the hollow spaces of the reservoir 20 and the supply unit 31 have another shape, for example, in the case where these hollow spaces have an angular cylindrical shape, the diameters and the like described below can be converted into the lengths in the direction of gravity (direction X), the widths and depths in the horizontal direction (direction Y), and the like.

The total volume of the reservoir 20 is, for example, 0.05 to 10 mL, the volume of the main body 203 is, for example, 0.03 to 9 mL, and the volume of the connection portion 204 is, for example, 0.01 to 1 mL. The volume of fluid 70 that is designed to be stored in the main body 203 of the reservoir 20 is, for example, 70% to 95% of the total volume of the main body 203, and a specific example is 0.1 to 1 mL. The total height H(r1) + H(r2) of the inside of the main body 203 of the reservoir 20 is, for example, 0.5 to 5 cm. In the main body 203, the height H(r1) from the bottom surface 201 to the designed fluid level position P(f) of the fluid 70 is, for example, 1/5 to 4/5 of the total height of the main body 203, and a specific example is 0.5 to 2 cm. In the main body 203 of the reservoir 20, the height H(r2) from the designed fluid level position P(f) to the top opening is, for example, 0.2 to 1.5 cm. The diameter D(r1) of the inside of the reservoir 20 is, for example, 0.2 to 2 cm. The diameter D(r2) of the communicating portion 202 in the bottom surface 201 is, for example, 1/10 to 1/2 of the diameter D(r1), and a specific example is 0.02 to 1 cm. The height H(r3) of the connection portion 204 of the reservoir 20 is, for example, 1/5 to 1/2 of the total height H(r1) + H(r2) of the main body 203, and a specific example is 0.5 to 2 cm.

The total length H(n) of the supply unit 31 is, for example, 1 to 4 cm. The length H(s) of the slit-like vent portion 314 is, for example, 1/2 to 4/5 of the total length H(n), and a specific example is 0.05 to 3.2 cm. The inner diameter D(n) of the supply unit 31 is, for example, 0.01 to 0.5 cm. The width W(s) of the vent portion 314 is, for example, 1/5 to 1/2 of the circumference of the supply unit 31, and a specific example is 0.002 to 0.25 cm. The width W(s) of the vent portion 314 of the supply unit 31 may be, for example, the length of an arc along the circumference or the length of the linear distance in the direction Y Note that there is no particular limitation on the size of the vent portion 314, and, for example, the vent portion 314 can have any size that allows bubbles in the supply unit 31 to exit in a lateral direction (direction Y) through the vent portion 314 in the side surface portion.

The lower end of the vent portion 314 of the supply unit 31 is, for example, preferably away from the communicating portion 202 of the reservoir 20 by a certain distance or more, and this distance H(d) is, for example, 0.1 to 1 cm. In addition, the outlet port 313 of the supply unit 31 is, for example, preferably away from the communicating portion 202 of the reservoir 20 by a certain distance or more, and this distance H(d) is, for example, 0.1 to 1 cm. In the direction of gravity, the position of the communicating portion 202 of the reservoir 20 can also be said to be at the bottom surface (upper surface of the bottom portion 201) of the reservoir 20, for example.

The ratio (R/T) of the cross-sectional area (R) of the reservoir 20 to the cross-sectional area (T) of the supply unit 31 is, for example, 1.5 or more, or 2 or more, because in this case bubbles coming out of the vent portion 314 of the supply unit 31 are more likely to move upward in the reservoir 20. The upper limit of the ratio (R/T) is not particularly limited, and is 24 or less, 20 or less, 10 or less, or 6 or less, for example. The cross-sectional area (T) of the supply unit 31 and the cross-sectional area (R) of the reservoir 20 are the cross-sectional areas of their respective hollow spaces, or specifically, for example, the areas of horizontal cross sections passing through the end portion of the vent portion 314 on the inlet port 312 side.

Next, a method for delivering the fluid in the fluid handling system 1 will be described below by way of example using Fig. 1. First, the valve 15 in the branch flow channel 401 connected to the fluid chamber 51 is opened, and the fluid delivery unit is driven. Thus, the fluid in the fluid chamber 51 is caused to flow into the circulation flow channel 40 via the branch flow channel 401, and is delivered. The fluid flows into the reservoir 20 via the supply unit 31 connected to the circulation flow channel 40. Then, the fluid is stored in the reservoir 20 as a fluid layer (not shown), and is also caused to flow into (introduced into) the inlet port 102 of the fluid handling device 10 via the communicating portion 202 in the bottom portion 201 of the reservoir 20, flow through the first flow channel 101, and then flow out through the outlet port 103 of the fluid handling device 10 into the circulation flow channel 40. When the valve 15 of the branch flow channel 402 connected to the collection chamber 52 is closed, the fluid that has flowed out passes through the circulation flow channel 40 and is again delivered to the reservoir 20, the supply unit 31, and the fluid handling device 10 in this order, and thus circulated in the fluid handling system 1. In this case, the valve in the branch flow channel 401 connected to the fluid chamber 51 is, for example, opened if it is desired to cause an additional amount of the fluid to flow, or closed if it is desired to not cause an additional amount of the fluid to flow. On the other hand, when the fluid that has passed through the first flow channel 101 of the fluid handling device 10 is to be collected (or drained), for example, the valve 15 in the branch flow channel 402 connected to the collection chamber 52 can be opened to cause the fluid to flow into the collection chamber 52.

Note that, for example, the fluid may be stored in the reservoir 20 in advance, and afterward, the fluid stored in the reservoir 20 may be caused to flow into the fluid handling device 10 by driving the fluid delivery unit.

According to the present embodiment, the supply unit 31 has the slit-like vent portion 314 in the side surface portion, and therefore, bubbles in the supply unit 31 flow out of the supply unit 31 through the vent portion 314. In other words, in the fluid layer stored in the reservoir 20, bubbles in the supply unit 31 flow out through the vent portion 314 in the lateral direction (horizontal direction, direction Y), rise in the fluid layer 70, and are then released from the fluid layer 70 into the atmosphere (also called an air layer) above the fluid layer 70.

In the fluid handling system of the present embodiment, it is preferable that the reservoir is a closed system, for example, during use, or specifically, while the fluid is being delivered. The expression "the reservoir is a closed system" means that, for example, the inside of the reservoir is closed from the outside, except for communication with the supply unit and communication with the fluid handling device. The closed system may be, for example, in a liquid-tight state, that is, a state in which the fluid in the reservoir is prevented from leaking out or the fluid outside the reservoir is prevented from entering the reservoir, or may be even in an airtight state, that is, a state in which a gas in the reservoir is prevented from leaking out or a gas outside the reservoir is prevented from entering the reservoir. The closed system of the reservoir may be achieved by, for example, the structure of the reservoir itself or by another configuration. The closed system will be described later in other embodiments.

### (Embodiment 2)

The present embodiment shows an example in which the vent portion of the supply unit is a through hole. The supply unit of the present embodiment has a form in which the entire vent portion is located above the outlet port of the supply unit (i.e., located above and away from the outlet port). The description of Embodiment 1 above can be applied to the present embodiment unless otherwise stated.

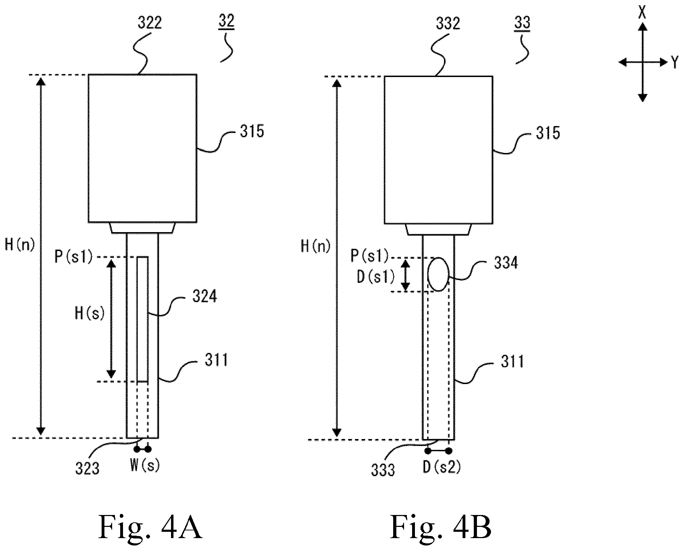

Figs. 4A and 4B show examples of the supply unit of the present embodiment. Fig. 4Ais a side view of a supply unit 32, and Fig. 4B is a side view of a supply unit 33. In Figs. 4A and 4B, the direction X is the direction of gravity or the axial direction, and the direction Y is the horizontal direction or a direction that is orthogonal to the direction X.

In the supply unit 32 in Fig. 4A, the main body 311 has a hollow space that constitutes the second flow channel, and openings at both ends of the second flow channel constitute an inlet port 322 and an outlet port 323. A side surface portion of the main body 311 of the supply unit 32 has an angular through hole 324 as the vent portion for the second flow channel. The shape of the through hole 324 may be, for example, a quadrangle such as a square or a rectangle, or another polygon, and, as illustrated in Fig. 4A, a rectangle is preferable.

As in the case of Embodiment 1 described above, the supply unit 32, which has the through hole as the vent portion 324, is accommodated in the reservoir such that the entire vent portion 324 is located above and away from the first flow channel communicating portion 202 of the reservoir 20. Furthermore, it is preferable that the supply unit 32 is disposed such that, for example, an end portion P(sl) of the vent portion 324 on the inlet port 322 side is located above the designed fluid level position P(f) in the reservoir 20. For example, the longer the length H(s) of the vent portion 324, the higher the versatility with which the vent portion 324 can be used when the position of the fluid level of the fluid stored in the reservoir 20 rises or lowers.

The total length H(n) of the supply unit 32 is, for example, 1 to 4 cm. The length H(s) of vent portion 324 is, for example, 1/2 to 4/5 of the total length H(n), and a specific example is 0.05 to 3.2 cm. The inner diameter D(n) of the supply unit 32 is, for example, 0.01 to 0.5 cm. The width W(s) of the vent portion 324 is, for example, 1/5 to 1/2 of the circumference of the supply unit 32, and a specific example is 0.002 to 0.25 cm. The width W(s) of the vent portion 324 of the supply unit 32 may be, for example, the length of an arc along the circumference or the length of the linear distance in the direction Y Note that there is no particular limitation on the size of the vent portion 324, and, for example, the vent portion 324 can have any size that allows bubbles in the supply unit 32 to exit in the lateral direction (direction Y) through the vent portion 324 in the side surface portion.

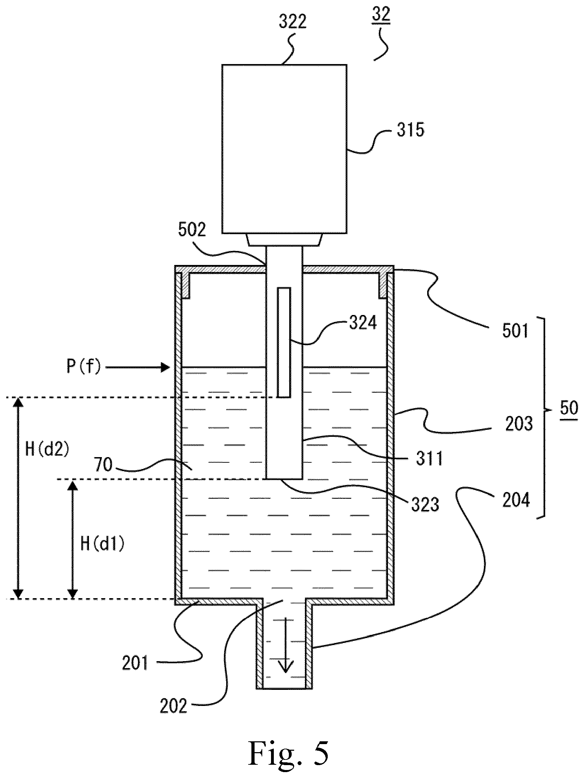

The distance (H(d1) in Fig. 5, which will be described later) between the outlet port 323 of the supply unit 32 and the communicating portion 202 of the reservoir 20 is, for example, 0.1 to 1 cm. In addition, the distance (H(d2) in Fig. 5, which will be described later) between the lower end of the vent portion 324 of the supply unit 32 and the communicating portion 202 of the reservoir 20 is, for example, 0.2 to 1.5 cm. In the direction of gravity, the position of the communicating portion 202 of the reservoir 20 can also be said to be at the bottom surface (upper surface of the bottom portion 201) of the reservoir 20, for example.

Next, in the supply unit 33 shown in Fig. 4B, the main body 311 has a hollow space that constitutes the second flow channel, and openings at both ends of the second flow channel constitute an inlet port 332 and an outlet port 333. A side surface portion of the main body 311 of the supply unit 33 has a circular through hole 334 as the vent portion for the second flow channel. The shape of the through hole 334 may be, for example, a circular shape such as a perfect circle or an ellipse, and, as illustrated in Fig. 4B, an ellipse is preferable.

The total length H(n) of the supply unit 33 is, for example, 1 to 4 cm. The major axis D(s1) of the vent portion 334 in the direction X is, for example, 1/2 to 4/5 of the total length H(n), and a specific example is 0.05 to 3.2 cm. The inner diameter D(n) of the supply unit 33 is, for example, 0.01 to 0.5 cm. The minor axis D(s2) of the vent portion 334 in the horizontal direction (direction Y) is, for example, 1/5 to 1/2 of the circumference of the supply unit 33, and a specific example is 0.002 to 0.25 cm. The minor axis D(s2) of the vent portion 334 of the supply unit 33 may be, for example, the length of an arc along the circumference or the length of the linear distance in the horizontal direction. Note that there is no particular limitation on the size of the vent portion 334, and, for example, the vent portion 334 can have any size that allows bubbles in the supply unit 33 to exit in the lateral direction (direction Y) through the vent portion 334 in the side surface portion.

As in the case of Embodiment 1 described above, the supply unit 33, which has the through hole as the vent portion 334, is accommodated in the reservoir 20 such that the entire vent portion 334 is located above and away from the first flow channel communicating portion 202 of the reservoir 20. Furthermore, it is preferable that the supply unit 33 is disposed such that, for example, an end portion P(sl) of the vent portion 334 on the inlet port 332 side is located above the designed fluid level position P(f) in the reservoir 20. Furthermore, for example, the longer the major axis D(s1) of the vent portion 334, the higher the versatility with which the vent portion 334 can be used when the position of the fluid level in the reservoir 20 rises or lowers.

### (Embodiment 3)

In the present embodiment, an example in which the reservoir further has a lid portion will be described. The description of the foregoing embodiments can be applied to the present embodiment unless otherwise stated.

Fig. 5 shows a schematic diagram of a reservoir with a lid portion in which the supply unit is accommodated. In Fig. 5, the supply unit 32 shown in Fig. 4A of Embodiment 2 above is illustrated as the supply unit, but the supply unit is not limited to this. In addition, Fig. 5 is a partial cross-sectional view, and shows a cross-sectional view of a reservoir 50 and a side view of the supply unit 32 for the sake of convenience.

As is the case with the reservoir 20 in Fig. 3 of Embodiment 1, the reservoir 50 has the main body 203 that is open at the top and the connection portion 204 and further has a lid portion 501. The lid portion 501 is disposed so as to cover the top opening of the main body 201. For example, the lid portion 501 may be integrally molded with the main body 203 and the connection portion 204, or may be a separate member that is placed on the main body 203 during use. The lid portion 501 has a through hole 502, for example, and the supply unit 32 is inserted into the through hole 502. The lid portion 501 is not particularly limited, and, for example, may be a liquid-tight lid portion or a non-liquid-tight lid portion or may be an airtight lid portion or a non-airtight lid portion.

The lid portion 501 is preferably, for example, a lid portion that makes the inside of the reservoir 50 a closed system. By making the inside of the reservoir 50 a closed system with the lid portion 501, it is possible to prevent contamination of the fluid stored in the reservoir 50 and the fluid that has circulated through the fluid handling system, evaporation of the fluid in the reservoir 50, and the like. In this case, the lid portion 501 is, for example, preferably a liquid-tight lid portion or an airtight lid portion as described above, and more preferably an airtight lid portion.

For example, the lid portion 501 may be an imperforate member that is capable of completely sealing the inside of the reservoir 50, or a member that has a hole for releasing the internal pressure according to the desired level of prevention of the contamination and evaporation. As the latter, for example, a porous member or the like can also be used.

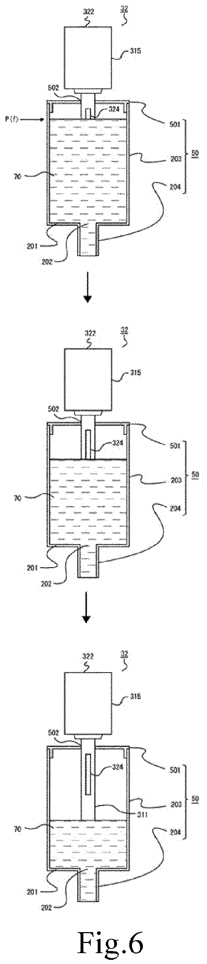

In the case where the lid portion 501 is a member that is capable of sealing the inside of the reservoir 50 in an airtight manner, the volume of the reservoir 50 is preferably set as follows, for example. That is, the volume of the reservoir 50 is preferably equal to or greater than the total volume of a gas expected to enter the reservoir 50 while the fluid is circulating through the fluid handling system. In addition, it is preferable that the inlet port 322-side end portion of the vent portion 324 of the supply unit 32 is located at approximately the same position as a surface of the lid portion 501 on the inner side of the reservoir 50, and it is preferable that the designed fluid level position P(f) in the reservoir 50 is located at 80% or more, 90% or more, or 95% or more of the total volume of the main body 203 of the reservoir 50. By setting such conditions, it is possible to obtain the following additional effects, for example. The effects will be described using a schematic diagram of Fig. 6. Fig. 6 is a schematic diagram showing the relationship between the reservoir 50 and the supply unit 32, as in Fig. 5, and shows changes in the position of the fluid layer 70 during use. In addition, Fig. 6 is a partial cross-sectional view, and shows a cross-sectional view of the reservoir 50 and a side view of the supply unit 32 for the sake of convenience.

Bubbles entering the supply unit 32 along with the fluid 70 flow out of the supply unit 32 through the vent portion 324 into the fluid layer 70 in the reservoir 50, rise in the fluid layer 70, and are then released into the air layer above the fluid layer 70. In this case, when the lid portion 501 is a member capable of sealing the inside of the reservoir 50 in an airtight manner, for example, the gas that has passed through the fluid layer 70 is not discharged to the outside of the reservoir 50 and accumulates on the fluid layer 70, and accordingly, the fluid level of the fluid layer 70 lowers toward the fluid handling device 10 as shown in the left figure to the center figure in Fig. 6. However, in the present embodiment, as a result of setting the volume of the reservoir 50, the position of the vent portion 324, the designed fluid level position P(f), and the like as described above, for example, even when the gas accumulates on the fluid layer 70 and the fluid level of the fluid layer 70 lowers, the gas accumulating in the reservoir 50 can be prevented from coming into contact with the inlet port 102 of the fluid handling device 10, as shown in the right figure in Fig. 6, and thus, the gas can be sufficiently prevented from entering the first flow channel 101 of the fluid handling device 10.

### (Embodiment 4)

In the present embodiment, for example, an example will be described in which the reservoir is made a closed system not by the structure of the reservoir described in Embodiment 3 above, but by another configuration during use. Specifically, the present embodiment is an example in which the fluid in the reservoir is made a closed system by forming, in the reservoir, a layer of a non-affinity solvent that has no affinity for the fluid. The description of the foregoing embodiments can be applied to the present embodiment unless otherwise stated.

For example, a solvent that separates from the fluid and has a lower specific gravity than the above-described aqueous solvent can be used as the non-affinity solvent. When the fluid is the aqueous solvent, an example of the non-affinity solvent is an oily solvent, and an oil such as mineral oil can be used as the oily solvent.

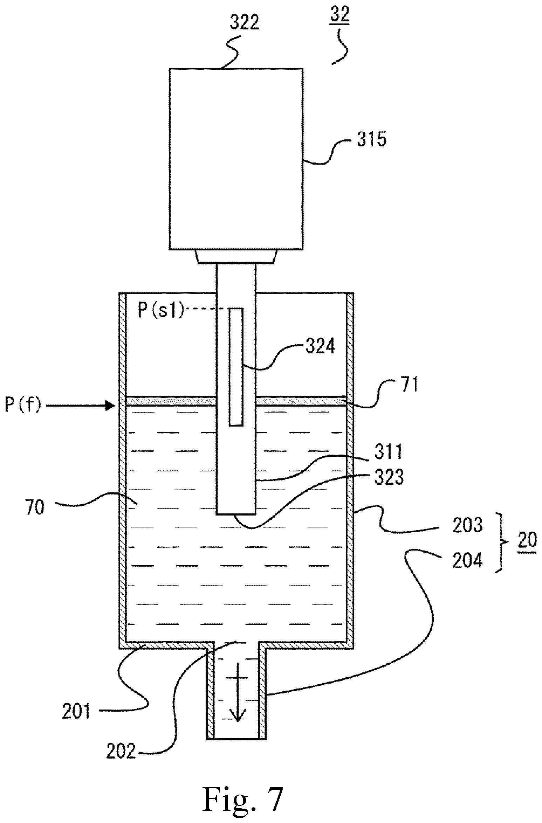

Fig. 7 shows a schematic diagram in which the supply unit is accommodated in the reservoir, and the fluid in the reservoir is made a closed system by a non-affinity solvent layer. In Fig. 7, the supply unit 32 shown in Fig. 4A of Embodiment 2 above is illustrated as the supply unit, but the supply unit is not limited to this. In addition, Fig. 7 is a partial cross-sectional view, and specifically, shows a side view of the supply unit 32 while showing a cross-sectional view of the reservoir 20.

The reservoir 20 is the same as the reservoir 20 in Fig. 3 of Embodiment 1 above and is open at the top. In this form, for example, the outlet port 323 side of the supply unit 32 is first accommodated in the reservoir 20, and an aqueous solvent is supplied to the reservoir 20 as the fluid 70 using a pipette or the like, without driving the fluid delivery unit. Subsequently, an oil is supplied onto the fluid (aqueous solvent) layer 70 in the reservoir 20 using a pipette or the like. Since the oil separates from the aqueous solvent and has a lower specific gravity than the aqueous solvent, an oil layer 71 is formed on the fluid layer 70. Thus, although the reservoir 20 does not have a lid portion such as that described in Embodiment 3 above, the fluid 70 inside can be made a closed system. Then, the fluid can be delivered to the fluid handling system 1 by driving the fluid delivery unit.

When the upper end portion P(sl) of the vent portion 322 of the supply unit 32 is located above the oil layer 71 as shown, for example, in Fig. 7, bubbles in the supply unit 32 pass through the vent portion 324, for example, and are released into the atmosphere above the oil layer 71. When the upper end portion P(sl) of the vent portion 324 of the supply unit 32 is located in the oil layer 71 or the fluid layer 70 in the reservoir 20 as well, bubbles in the supply unit 32 pass through the vent portion 32 and are released into the oil layer 71 or the fluid layer 70, but the bubbles further rise and pass through the oil layer 71, and are then released into the atmosphere outside.

According to the present embodiment, as in the case of Embodiment 3 above, it is possible to prevent contamination of the fluid stored in the reservoir 20 and the fluid that has circulated through the fluid handling system, evaporation of the fluid in the reservoir 20, and the like.

Furthermore, since the reservoir 20 is open at the top, for example, even when the amount of the fluid under the oil layer 71 increases or decreases, the oil layer 71 can also move up or down accordingly. Therefore, for example, even if air expansion or the like occurs in the fluid handling system, the pressure caused by the air expansion can be released by the oil layer 71 moving up or down.

Furthermore, since the reservoir 20 is open at the top, for example, the fluid that has passed through the first flow channel 101 of the fluid handling device 10 and returned to the reservoir 20 can also be easily sampled from the inside of the reservoir 20 using a pipette or the like.

There is no particular limitation on the volume ratio between the stored fluid 70 and the non-affinity solvent 71 in the reservoir 20, and, when the volume of the fluid 70 is taken as 1, the volume of the non-affinity solvent is 1/2 to 1/10, for example.

### (Embodiment 5)

A fluid handling system of the present embodiment has a form in which the fluid is caused to flow into the reservoir from below the reservoir, and an example of this form is a form in which the supply unit is accommodated in the reservoir while extending upward from the bottom of the reservoir. The description of the foregoing embodiments can be applied to the present embodiment unless otherwise stated.

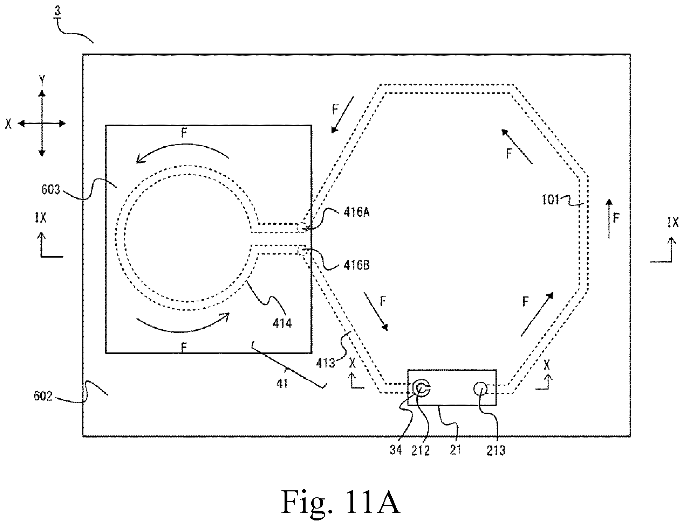

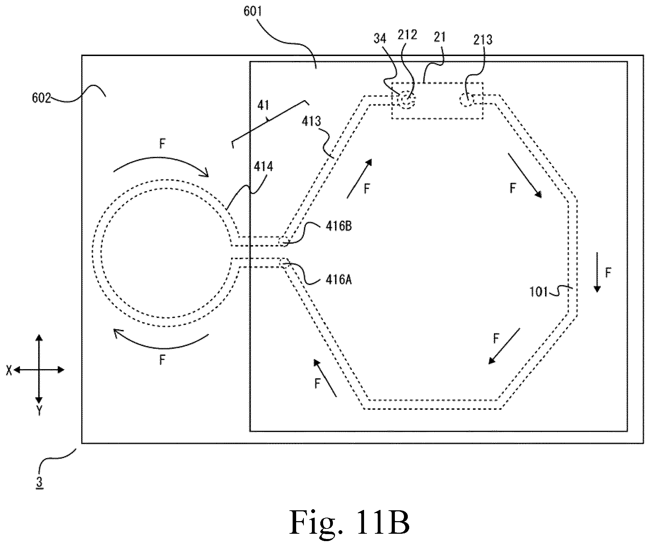

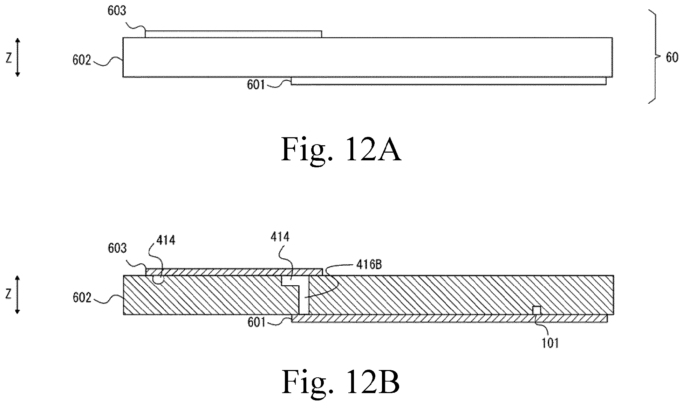

Examples of the fluid handling system of the present embodiment and a partial configuration thereof are shown in schematic diagrams of Figs. 11A to 14. Figs. 11A, 11B, 12A, and 12B are schematic diagrams showing a fluid handling system 3. Fig. 11A is a plan view (top view) of the fluid handling system 3 as seen from an upper surface side, and Fig. 11B is a plan view (bottom view) of the fluid handling system 3 as seen from a back surface side. Fig. 12Ais a side view of the fluid handling system 3, and Fig. 12B is a cross-sectional view of the fluid handling system 3 in Figs. 11A and 11B taken in direction IX-IX. In Figs. 11A and 11B, arrows F indicate the flow direction of the fluid.

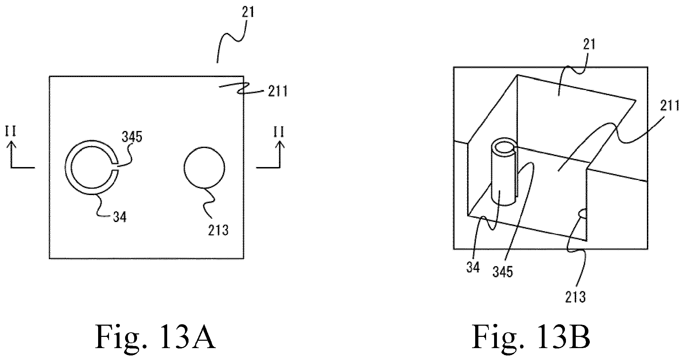

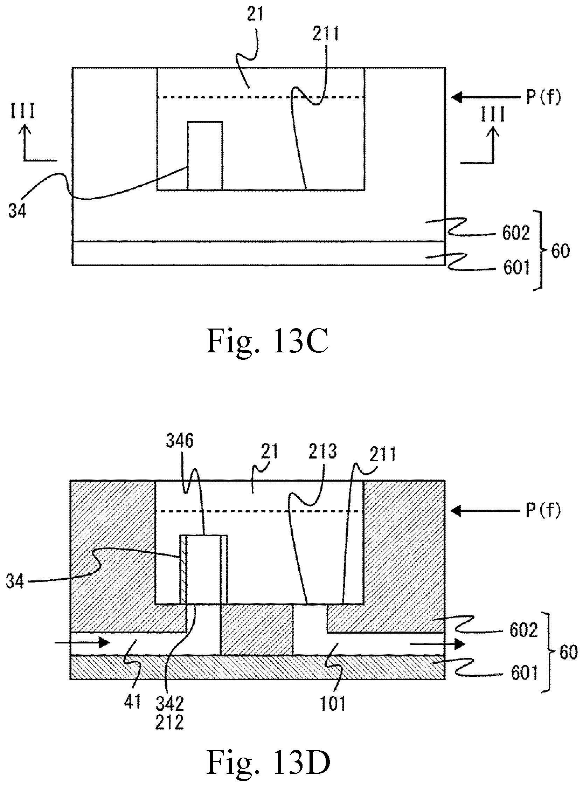

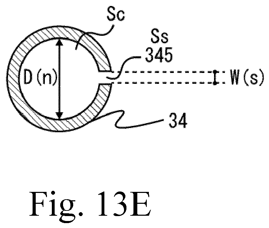

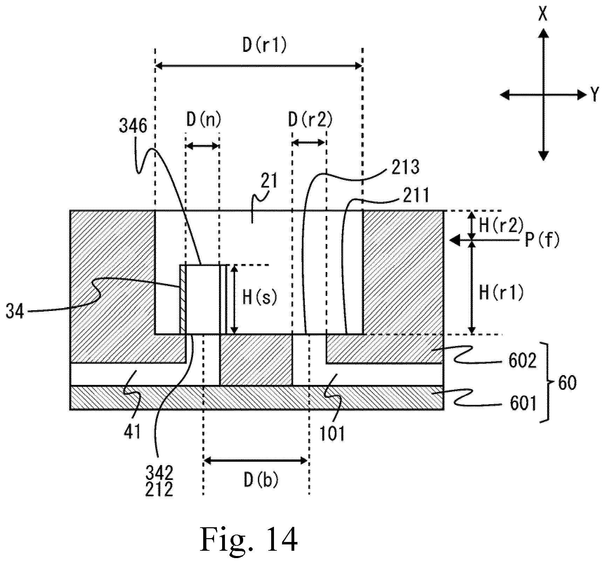

Figs. 13A to 13E and 14 are schematic diagrams showing a reservoir 21 with a supply unit 34, extracted from the fluid handling system 3. Fig. 13A is a plan view (top view) of the reservoir 21 as seen from an upper surface side, Fig. 13B is a perspective view of the inside of the reservoir 21, Fig. 13C is a side view of the inside of the reservoir 21, Fig. 13D is a cross-sectional view of the reservoir 21 in Fig. 13A taken in direction II-II, and Fig. 13E is a cross-sectional view of the supply unit 34 in Fig. 13C taken in direction III-III. Figs. 13B and 13C both show the inside of the reservoir 21 with one lateral side (paper surface side in the figures) thereof omitted.

In Figs. 11A to 12, X and Y indicate directions (horizontal directions) that are orthogonal to each other in a plane, and Z indicates a direction (direction of gravity) that is orthogonal to the directions X and Y and also referred to as the "thickness direction".

The fluid handling system 3 has the first flow channel 101 of the fluid handling device, the reservoir 21, the supply unit 34, and a circulation flow channel 41. The circulation flow channel 41 has a contact region 414 that comes into contact with the fluid delivery unit (not shown) and a downstream region 413 that is connected to the reservoir 21, the downstream region 413 being located downstream of the contact region 414 in the flow direction of the fluid. The first flow channel 101 is connected to the reservoir 21 at an upstream end thereof and connected to the circulation flow channel 41 at a downstream end thereof.

In the fluid handling system 3, the reservoir 21, the first flow channel 101, and the circulation flow channel 41 will also be referred to collectively as a circulation route for the first flow channel 101.

In the fluid handling system 3, a main body 60 has the first flow channel 101, the reservoir 21, the supply unit 34, and the circulation flow channel 41. Since the main body 60 has the first flow channel 101, the fluid handling system 3 has a form in which the main body 60 includes the fluid handling device. The main body 60 is a stacked body having a substrate 602, a cover 601, and a cover 603. The substrate 602 has, in one surface (upper surface in Figs. 11A and 11B) thereof, a recess that serves as the reservoir 21 and a recess that serves as the contact region 414 of the circulation flow channel 41. The substrate 602 further has through holes at both ends 416A and 416B of the contact region 414 and at two positions 212 and 213 in the recess serving as the reservoir 21, the through holes penetrating the substrate 602 to the other surface (lower surface in Figs. 11A and 11B) thereof. On the other hand, the substrate 602 has, in the other surface (lower surface) thereof, a recess that serves as the first flow channel 101 and a recess that serves as the downstream region 413 of the circulation flow channel 41. In the substrate 602, the contact region 414 of the circulation flow channel 41 is, in the vertical direction, in communication with the recess (downstream end) of the first flow channel 101 via the through hole at the end 416A, and in communication with the recess (downstream end) of the contact region 414 at the end 416B. In addition, in the substrate 602, one opening 212 of a bottom portion 211 of the reservoir 21 is, in the vertical direction, in communication with the recess (downstream end) of the circulation flow channel 41 via the through hole and serves as a circulation flow channel communicating portion, and the other opening 213 is in communication with the recess (upstream end) of the first flow channel 101 and serves as a first flow channel communicating portion. The opening 212 is also referred to as the circulation flow channel communicating portion, and the opening 213 is also referred to as the first flow channel communicating portion.

Moreover, on the upper surface of the substrate 602, the recess serving as the reservoir 21 is exposed, and the cover 603 that covers the recesses serving as the first flow channel 101 and the contact region 414 is disposed, while on the lower surface of the substrate 602, the cover 601 that covers the recesses serving as the first flow channel 101 and the downstream region 413 is disposed. With this structure, the contact region 414 of the circulation flow channel 41 and the reservoir 21 that is open at the top are formed on the upper surface side of the substrate 602, and the first flow channel 101 and the downstream region 413 of the circulation flow channel 41 are formed on the lower surface side of the substrate 602.

Inside the reservoir 21, the opening of the through hole that communicates with the circulation flow channel 41 is the communicating portion 212 for the circulation flow channel 41 and serves as an inlet port through which the fluid flows into the reservoir 21, and the opening of the through hole that communicates with the first flow channel 101 is the communicating portion 213 for the first flow channel and serves as an outlet port through which the fluid flows out of the reservoir 21.

In addition, the supply unit 34 protruding upward is disposed on the communicating portion 212 (inlet port) of the reservoir 21. The supply unit 34 is a hollow cylindrical body and has a slit 345 extending in the direction of gravity (vertical direction) in its side surface portion. In the supply unit 34 of the present embodiment, a lower opening 342 serves as the fluid inlet port, an upper opening 346 serves as the vent portion, and the slit 345 serves as the fluid outlet port.

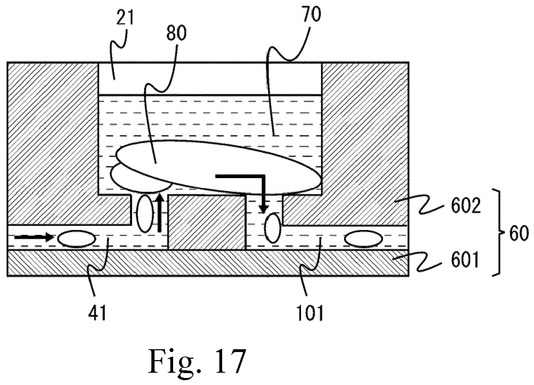

Here, in order to describe the effects of the fluid handling system of the present embodiment, an example of a reservoir without the supply unit is shown in Fig. 17 for comparison. Fig. 17 is a schematic diagram showing the state of bubbles in the reservoir and shows the same configuration as Figs. 11A and 11B of the present embodiment, except that the supply unit is not disposed. The reservoir 21 has, in its bottom portion 211, an inlet port through which the fluid flows into the reservoir 21 and an outlet port through which the fluid flows out of the reservoir 21. Therefore, when the fluid flows into the reservoir 21 from the circulation flow channel 41 via the inlet port, if a gas 80 is mixed in the fluid, the gas 80 may flow into the first flow channel 101 through the outlet port along with the fluid. Furthermore, if bubbles 80 continue flowing into the reservoir 21, the bubbles will coalesce and grow into a large bubble, increasing the likelihood of the gas 80 flowing into the first flow channel 101 through the outlet port of the reservoir 21. This problem is likely to occur, for example, when the volume of the inside of the reservoir 21 is relatively small, when the amount of fluid stored in the reservoir 21 is relatively small, or when the distance between the outlet port and the inlet port of the reservoir 21 is relatively small.

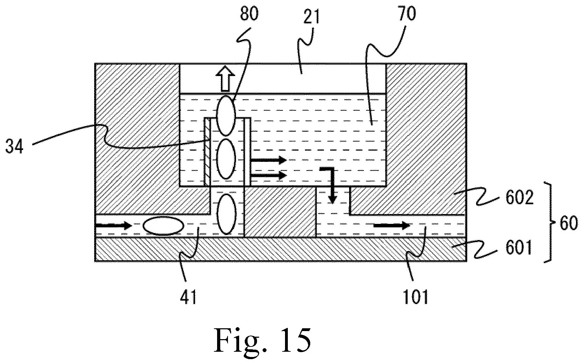

In contrast, the fluid handling system of the present embodiment is provided with the supply unit 34 having the slit 345, and thus achieves the following effects. A schematic diagram of Fig. 15 illustrates the state of bubbles in the reservoir 21 in which the supply unit 34 is disposed. That is, when fluid delivery is performed using the fluid handling system described above, for example, the fluid that has flowed through the circulation flow channel 41 is caused to flow into the supply unit 34 through the inlet port 342 of the supply unit 34 via the circulation flow channel communicating portion (inlet port) 212 of the reservoir 21. Since the supply unit 34 has the slit 345, the slit 345 serves as the fluid outlet port, and the fluid flows out of the supply unit 34 through the slit 345. When the fluid contains bubbles 80, since the bubbles 80 are lighter than the fluid, the bubbles 80 are guided by the supply unit 34 extending upward and rise in the supply unit 34, and as a result, the gas is released through the opening at the upper end of the supply unit 34, the opening serving as the vent portion 346. In addition, when the fluid is stored to the position P(f) in the reservoir 21, the gas 80 released through the vent portion 346 at the upper end of the supply unit 34 passes through the fluid layer above the vent portion 346 and is released into the atmosphere (white arrow). On the other hand, the fluid stored in the reservoir 21 is caused to flow into the first flow channel 101 through the other communicating portion (outlet port) 213 for the first flow channel of the reservoir 21 (black arrows). As described above, even when the fluid flowing from the circulation flow channel 41 contains bubbles, the bubble is guided by the supply unit 34 and released upward, and therefore, it is possible to prevent the gas from flowing out through the first flow channel communicating portion (outlet port) 213 of the reservoir 21 into the first flow channel 101.

Now, the fluid handling system of the present embodiment will be described in greater detail.

The supply unit 34 is a hollow tube, and the hollow space (also referred to as "internal space") thereof constitutes the second flow channel. An opening at a lower end of the tube constitutes the inlet port 342 through which the fluid flows into the inside of the tube, and an opening at an upper end of the tube constitutes the vent portion 346 through which a gas flows out. The side surface portion (also referred to as "wall portion") of the supply unit 34 has the slit 345, and the slit 345 constitutes the outlet port through which the fluid flows out. In the present embodiment, the slit 345 is a cut that is formed extending from the lower end opening (inlet port) 342 to the upper end opening (vent portion) 346. There is no particular limitation on the shape of the hollow space of the supply unit 34, and, for example, the illustration in Embodiment 1 above can be applied thereto.

In the supply unit 34, the outlet port (slit 345) is located above the inlet port (lower end opening 342), and the vent portion (upper end opening 346) is located above the inlet port (lower end opening 342) and the outlet port (slit 345). Furthermore, it is preferable that an end portion (lower end) of the outlet port (slit 345) of the supply unit 34 on the inlet port (opening 342) side is located below the designed fluid level position P(f) in the reservoir 21, for example. An end portion (upper end) of the supply unit 34 on the vent portion (upper opening 346) side may be located above the designed fluid level position P(f) in the reservoir 21, but it is preferable that this end portion is located below the designed fluid level position P(f) in the reservoir 21. In this case, it can also be said that the entire outlet port (slit 345) of the supply unit 34 is located below the designed fluid level position P(f).

The slit 345 of the supply unit 34 in Figs. 13 and 14 is formed extending from the lower end opening 342 (position of the communicating portion 212 of the reservoir 21) to the upper end opening, of the supply unit 34. In the present embodiment, the slit 345 of the supply unit 34 serves as the outlet port through which the fluid that has flowed into the supply unit 34 flows to the outside. Therefore, for example, it is preferable that the lower end of the slit 345 is located at the same position as the lower end opening 342 of the supply unit 34 (position of the communicating portion 212 of the reservoir 21). In addition, the position of the upper end of the slit 345 is not particularly limited, and, for example, the upper end of the slit 345 may be located at the position of the upper end opening 346 of the supply unit 34 or may be located at a position between the lower end opening 342 and the upper opening 346.

In Figs. 13 and 14, the opening serving as the vent portion 346 is shown at the upper end of the supply unit 34 by way of example, but the position of this opening is not limited to this. The opening serving as the vent portion may also be formed in, for example, the side surface portion of the supply unit 34. In that case, it is preferable that the opening serving as the vent portion is formed in a region of the side surface portion of the supply unit 34 on the upper end side, because bubbles in the fluid are easily guided and released into the atmosphere.

It is sufficient that the reservoir 21 can store the fluid as described above, and there is no particular limitation on the shape of the reservoir 21. As a specific example, the inside of the reservoir 21 may have a bottomed cylindrical shape as described above. The internal space of the reservoir 21 may have a circular shape or an angular shape in horizontal cross section, for example. In addition, the horizontal cross section of the internal space of the reservoir 21 may have, for example, the same shape with a constant size in the direction of gravity, or a shape that becomes smaller or larger downward. The communicating portions 212 and 213 in the bottom portion 211 of the reservoir 21 may have, for example, a circular shape or an angular shape. As shown in Figs. 11A and 11B, the internal structure of the reservoir 21 has, for example, a bottomed circular cylindrical shape (preferably a perfect circular cylindrical shape), and the communicating portions 212 and 213 in the bottom portion 211 have a circular shape (preferably a perfect circle).

In the reservoir 21, the positions of the communicating portions 212 and 213 are not particularly limited, but are preferably in the bottom portion 211 as shown in Figs. 13 and 14.

Figs. 13 and 14 show an example in which the communicating portion 212 of the reservoir 21 is in direct communication with the circulation flow channel 41 and the communicating portion 213 of the reservoir 21 is in direct communication with the first flow channel 101, but the present invention is not limited to this. For example, the communicating portion 212 of the reservoir 21 may have a connection portion and be in communication with the circulation flow channel 41 via the connection portion, and the communicating portion 213 of the reservoir 21 may have a connection portion and be in communication with the first flow channel 101 via the connection portion. In Figs. 13 and 14, for example, the flow channel downwardly extending from the communicating portion 212 of the reservoir 21 may be the connection portion, and the flow channel downwardly extending from the communicating portion 213 of the reservoir 21 may be the connection portion. The inside of each connection portion described above may have, for example, a cylindrical shape, and the hollow space of each connection portion may have a circular shape or an angular shape in horizontal cross section.

As shown in Figs. 13A to 13E, the supply unit 34 is disposed such that the entire supply unit 34 is located below the designed fluid level position P(f) in the reservoir 21, but the present invention is not limited to this. That is, when the fluid is stored in the reservoir 21, the vent portion 346 (upper opening) of the supply unit 34 may be located in the fluid layer or may be located above the fluid layer (in the air layer).

The substrate 602 is, for example, a molded body whose shape does not deform during use of the fluid handling system 3, and is preferably a hard substrate, and the material of the substrate 602 is a resin, for example. There is no particular limitation on the type of the resin. The cover 601 that covers the recesses in the lower surface of the substrate 602 is, for example, a resin cover, and may be hard, soft, or elastic. There is no particular limitation on the thickness of the cover 601, and the cover 601 may be a film or a sheet, for example. The cover 603 that covers the recess of the contact region 414 of the circulation flow channel 41 is, for example, a resin cover, and may be hard, soft, or elastic. There is no particular limitation on the thickness of the cover 603, and the cover 603 may be a film or a sheet, for example.

For fluid delivery in the fluid handling system, as described above, the fluid delivery unit, such as a roller pump, a rotary pump, or a pressure pump, can be used. When the main body 60 has the circulation flow channel 41 as in the present embodiment, it is preferable that, for example, the contact region 414 of the circulation flow channel 41 that comes into contact with the fluid delivery unit is soft, or preferably elastic. As a specific example, at least the cover 603 that covers the recess serving as the contact region 414, of the recesses of the substrate 602, is, for example, a soft cover, or preferably an elastic cover. For example, when the fluid delivery unit is the aforementioned rotary pump, the pressing portion (e.g., the sliding portion) can be brought into contact with and pressed against the contact region 414 of the circulation flow channel 41 from the upper surface side (i.e., the cover 603 side) of the main body 60 while being moved (e.g., slid) in the fluid delivery direction. As a result, a suction side (first flow channel 101 side) of the contact region 414 is depressurized and draws up the fluid, and a portion of the contact region 414 on which the pressing portion moves is pressurized and discharges the fluid. In this manner, the fluid is drawn and discharged in the circulation flow channel 41, and thus, the fluid can be circulated through a circulation route including the circulation flow channel 41, the supply unit 34, the reservoir 21, and the first flow channel 101.

Next, with respect to the fluid handling system of the present embodiment, the sizes of the reservoir 21 and the supply unit 34 will be described by way of example; however, their sizes are not limited to the examples below. Also, the sizes of the other components of the fluid handling system are not particularly limited and can be set as appropriate according to, for example, the sizes of the reservoir 21 and the supply unit 34. Hereinafter, the sizes of the reservoir 21 and the supply unit 34 will be described taking as an example a case in which both the inside of the reservoir 21 and the inside of the supply unit 34 have a circular cylindrical shape; however, the present invention is not limited to this. Furthermore, in the case where the inside of the reservoir 21 and the inside of the supply unit 34 have another shape, for example, in the case where they have an angular cylindrical shape, the diameters and the like described below can be converted into the lengths in the direction X of gravity, the widths and depths in the horizontal direction Y, and the like.

The total volume of the reservoir 21 is, for example, 0.05 to 10 mL. The volume of fluid that is designed to be stored in the reservoir 21 is, for example, 70% to 95% of the total volume of the reservoir 21, and a specific example is 0.1 to 1 mL. The height H(r1) + H(r2) of the inside of the reservoir 21 is, for example, 0.5 to 3 cm. The height H(r1) from the bottom surface 211 to the designed fluid level position P(f) is, for example, 1/5 to 4/5 of the total height of the inside of the reservoir 21, and a specific example is 0.1 to 2.4 cm. In the reservoir 21, the height H(r2) from the designed fluid level position P(f) to the top opening is, for example, 0.1 to 2.9 cm. The inner diameter D(r1) of the reservoir 21 is, for example, 0.15 cm. The diameters D(n) and D(r2) of the communicating portions 212 and 213 in the bottom surface 211 are, for example, 1/5 to 4/5 of the diameter D(r1), and a specific example is 0.03 to 0.12 cm. The distance D(b) between the centers of the two communicating portions 212 and 213 of the reservoir 21 is, for example, 0.2 to 1 cm.

The total length (in the direction of gravity) H(s) of the supply unit 34 is, for example, 0.3 to 1.2 cm, and the length of the slit 345 (outlet port) is equal thereto. The width (in the horizontal direction) W(s) of the slit 345 is, for example, 1/5 to 1/2 of the circumference of the supply unit 34, and a specific example is 0.02 to 0.5 cm. The width W(s) of the slit 345 may be, for example, the length of an arc along the circumference or the length of the linear distance in the horizontal direction. The inner diameter D(n) of the supply unit 34 is, for example, 0.02 to 0.8 cm.

In the supply unit 34, the ratio between the cross-sectional area (Sc) of a cross section of the hollow space that passes through the center of the supply unit 34 and the cross-sectional area (Ss) of the space of the slit 345 preferably satisfies Sc > Ss, because, for example, bubbles in the supply unit 34 are likely to be guided upward. The ratio of Sc to Ss (Sc/Ss) is, for example, 8 or more, or 16 or more. The upper limit of the ratio (Sc/Ss) is not particularly limited, and is, for example, 80 or less, 70 or less, 50 or less, 40 or less, 30 or less, or 20 or less. The cross-sectional area (Sc) of the hollow space of the supply unit 34 and the cross-sectional area (Ss) of the space of the slit 345 are each the area of a horizontal cross section, for example.

Note that, in Figs. 11A and 11B, a section from the end 416A of the contact region 414 to the communicating portion 212 between the downstream region 413 and the reservoir 21 is shown as the circulation flow channel 41, but the present invention is not limited to this, and, for example, a region on the upstream side in the contact region 414 may be called an "upstream region" that is connected to the first flow channel 101.

The fluid handling system 3 may further include, for example, a temperature controller. For example, when cells are placed in the first flow channel 101, the cells can be cultured, or a temperature condition close to that of the human body can be realized, by controlling the temperature using the temperature controller.

In the fluid handling system of the present embodiment, for example, as in the case of Embodiment 1 above, it is preferable that the reservoir is a closed system during use, or specifically, while the fluid is being delivered. The description of the foregoing embodiments can be applied to the closed system.

Next, a method for delivering the fluid to the first flow channel 101 of the fluid handling system 3 will be described below by way of example.

First, the fluid 70 is supplied to the reservoir 21 using a pipette or the like, without driving the fluid delivery unit. Next, a non-affinity solvent that has no affinity for the fluid 70 is further added to the reservoir 21 to which the fluid 70 has been supplied. Thus, a non-affinity solvent layer (not shown) separate from the fluid layer 70 is formed on the fluid layer 70. Then, fluid delivery in the direction of arrows F in Figs. 11A and 11B is started by driving the fluid delivery unit. As a result, the fluid stored in the reservoir 21 is caused to flow from the reservoir 21 into the first flow channel 101, pass through the first flow channel 101, flow into the circulation flow channel 41, pass through the circulation flow channel 41, and return to the reservoir 21. These steps are repeated.

Note that, in the present embodiment, an example in which the first flow channel 101 and the reservoir 21 are connected to each other via the circulation flow channel is shown, but the present invention is not limited to this. That is to say, the fluid handling system may or may not circulate the fluid in the circulation route for the first flow channel. In the case where the fluid is not circulated in the circulation route for the first flow channel, for example, a form may be adopted in which, instead of the circulation flow channel 41 that connects the first flow channel 101 and the reservoir 21, a connecting flow channel that connects the inlet port 212 of the reservoir 21 and another fluid chamber (not shown) is provided. In this case, as a result of the fluid chamber that stores a desired fluid and the inlet port 212 of the reservoir 21 being made to communicate with each other by the connecting flow channel, the fluid is caused to flow from the fluid chamber into the reservoir 21, and the fluid stored in the reservoir 21 is caused to flow into the first flow channel 101. The fluid that has passed through the first flow channel 101 may be collected, for example, at the downstream outlet port of the first flow channel 101.

### (Embodiment 6)

In Embodiment 5 above, an example in which the fluid outlet port of the supply unit is a slit has been described, but the present invention is not limited to this. The fluid outlet port of the supply unit may have, for example, the shape of a through hole formed in the side surface portion of the supply unit. The description of Embodiment 5 above can be applied to the present embodiment unless otherwise stated.

The shape of the through hole is not particularly limited, and may be an angular shape or a circular shape, for example. The angular shape may be, for example, a quadrangle, such as a square or a rectangle, or another polygon. The circular shape may be, for example, a perfect circle, an ellipse, or the like.

When the outlet port of the supply unit is the through hole, there is no particular limitation on the size of the through hole, and the through hole can have any size that allows the fluid to flow out of the supply unit via the through hole. As described above, in the present embodiment, if bubbles are mixed in the fluid, it is sufficient that the gas can be guided by the supply unit and released through the vent portion in an upper portion of the supply unit, and the fluid can flow out through the outlet port that is located below the vent portion. For this reason, it is preferable that, in the supply unit, the entire through hole is located on the lower end side of the supply unit rather than the upper end side of the supply unit.

### (Embodiment 7)

Uses of the fluid handling system of the present invention are not particularly limited, and, for example, the fluid handling system can be used to deliver a fluid to an organ-on-a-chip that reproduces a biological function. As a specific example, according to the fluid handling system, for example, by forming a blood vessel model in the first flow channel of the fluid handling device and delivering a fluid containing various reagents and the like thereto, it is also possible to reproduce a biological function using the blood vessel model.

In the fluid handling system in Fig. 1, an example in which one fluid chamber 51 and one collection chamber 52 are connected has been described, but the present invention is not limited to this, and as many fluid chambers 51 as the number of types of fluids required may be connected. When a blood vessel model is formed in the first flow channel of the fluid handling device, a plurality of fluid chambers that store, for example, a coating agent for the first flow channel, an aqueous solvent such as a buffer solution, an organic solvent such as ethanol, a vascular cell fluid, a reagent, and the like may be provided. In addition, preferably, the fluid handling system 1 further has a temperature controller to control the temperature in the range of 4°C to 40°C, for example, in order to achieve a condition close to that of the human body when, for example, forming a blood vessel model.

In the fluid handling system in Fig. 1, there are no particular limitations on the shape and size of the fluid handling device 10. The size of the fluid handling device 10 is set according to, for example, the number, length, and the like of first flow channels 101 formed in the fluid handling device 10. There is no particular limitation on the shape of the hollow space inside the first flow channel 101 of the fluid handling device 10, and, for example, a cross section in a direction (direction of gravity) orthogonal to the flow direction has a circular shape, a semi-circular shape, or an angular shape. The circular shape is, for example, a perfect circle or an ellipse, and is preferably a perfect circle. The angular shape is, for example, a quadrangle, such as a square or a rectangle, or another polygon, and is preferably a quadrangle. The cross-sectional area of the hollow space of the first flow channel 101 is, for example, 500 to 640,000 µm². There are no particular limitations on the sizes of the inlet port 102 and the outlet port 103 of the first flow channel 101, and, for example, the cross-sectional area of each of the hollow spaces is, for example, 30,000 to 80,000,000 µm².

The first flow channel 101 of the fluid handling device 10 can be formed by, for example, forming a recessed groove in at least one of an upper substrate and a lower substrate and stacking the two substrates so that the substrates face each other. In addition, the inlet port 102 and the outlet port 103 of the first flow channel 101 of the fluid handling device 10 can be formed by forming holes communicating with the groove, in one of the substrates. Alternatively, the first flow channel 101 may also be formed by forming a recessed groove in a surface of a substrate and disposing a cover such as a film so as to cover the groove. In this case, the inlet port 102 and the outlet port 103 of the first flow channel 101 of the fluid handling device 10 can be formed by forming holes communicating with the groove, in a surface of the substrate that is opposite to the groove.

### [Supply Unit and Kit for Fluid Handling System]

A supply unit for a fluid handling system of the present invention is a supply unit for use in the fluid handling system of the present invention, and the description of the supply unit of the fluid handling system of the present invention can be applied thereto. Also, a kit for a fluid handling system of the present invention is a kit for use in the fluid handling system of the present invention, and the description of the supply unit and the reservoir of the fluid handling system of the present invention can be applied thereto.

### [Method for Delivering Fluid]

A method for delivering a fluid of the present invention can be performed by using the fluid handling system of the present invention. The description with respect to the fluid handling system of the present invention can be applied to the method for delivering a fluid of the present invention.

### [Examples]

### [Example 1]

In the present example, a fluid was introduced into the first flow channel of the fluid handling device via the reservoir using the supply unit 31 with the slit-like vent portion 314 shown in Figs. 2A to 2D, and the behavior of bubbles in the supply unit 31 was observed.

### (1-1)

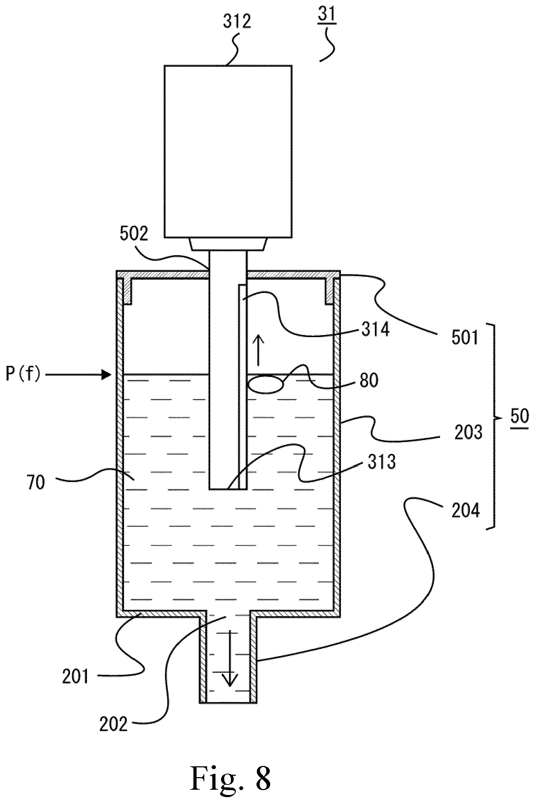

In the present example, as shown in Fig. 8, the closed-system reservoir 50 with the lid portion 501 shown in Fig. 5 was used as the reservoir, and the supply unit 31 shown in Figs. 2A to 2D was used. Also, the following system was constructed as a simplified fluid handling system similar to that shown in Fig. 1.

The circulation flow channel 40 in Fig. 1 was constituted by a substrate flow channel and two tubes. The substrate flow channel was formed by covering a recess in the substrate with an elastic film, like the contact region 414 in Figs. 11A and 11B. In the substrate flow channel, the hollow space of the flow channel had a semi-circular shape in cross section in a direction orthogonal to the fluid flow direction, and, the size of the flow channel was as follows: the inner radius was 6 mm, and the length in the fluid flow direction was 38 mm. Soft resin tubes with an inner diameter of 1 mm and a length of 15 cm were used as the two tubes.

The substrate flow channel (the contact region) of the circulation flow channel 40 was set under a rotary pump (trade name: Rotary Membrane Pump, available from Enplas). Then, one end of one of the tubes was connected to one end of the substrate flow channel, and the other end of that tube was connected to the outlet port 103 of the first flow channel 101 of the fluid handling device 10 shown in Fig. 1. A chip having a linear first flow channel 101 with a flow channel length of 18 cm was used as the fluid handling device 10. The supply unit 31 was inserted into the lid portion 501 removed from the reservoir 50, one end of the other tube was connected to the inlet port 312 of the supply unit 31, and the other end of the other tube was connected to the other end of the substrate flow channel.

Next, the leading end of the connection portion 204 of the reservoir 50 was connected to the inlet port 102 of the first flow channel 101 of the fluid handling device 10. Then, 1 mL of water containing a blue pigment was put into the reservoir 50 using a pipette until the position P(f) was reached. The lid portion 501 into which the supply unit 31 was inserted was fit into the top opening of the main body 203 of the reservoir 50 to make the inside of the main body 203 an airtight closed system.

The size of each member was as follows.
(1) Supply unit 31
   Total length H(n): 20 mm
   Length H(s) of slit 314: 12 mm
   Inner diameter D(n): 1 mm
   Width W(s) of slit 314: 0.5 mm
   Distance H(d) between leading end of supply unit 31 and bottom surface 201 of reservoir 50: 6 mm
(2) Reservoir 50
   Total volume: 1.5 mL
   Volume of main body 203: 1.4 mL
   Volume of connection portion 204: 0.06 mL
   Height H(r1) + H(r2) of main body 203: 20 mm
   Height H(r1) from bottom surface 201 to designed fluid level position P(f): 13 mm
   Height H(r2) from designed fluid level position P(f) to top opening: 7 mm.
   Inner diameter D(r1) of reservoir 50: 9 mm
   Diameter D(r2) of communicating portion 202 in bottom surface 201: 2 mm
   Height H(r3) of connection portion 204: 10 mm

Then, the pump was driven to circulate the water. The pump was driven under the conditions at a rotation speed of 16 RPM (supplied by a 12 V DC power supply).

As a comparative example, water was circulated in a similar manner using, instead of the supply unit 31 of the example above, the supply unit 30 shown in Fig. 16, which had the same size as the supply unit 31 and did not have the slit 314.

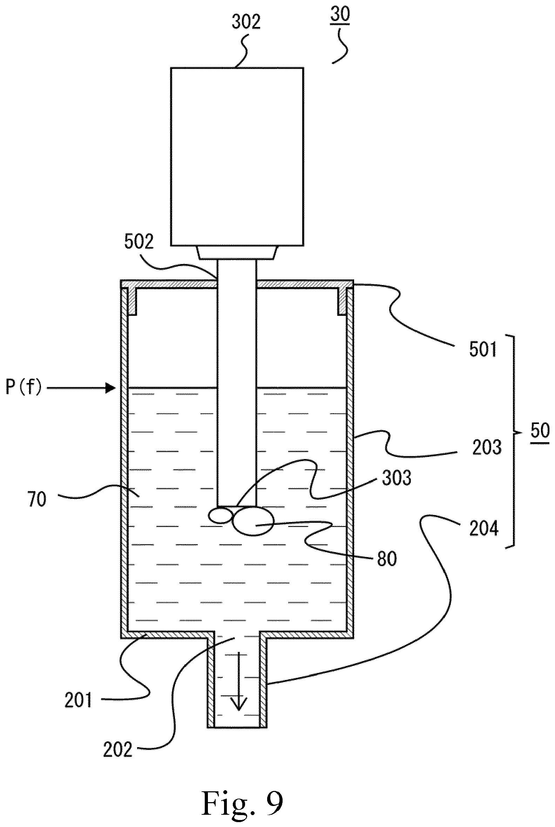

The results will be described using Figs. 8 and 9. Fig. 8 is a schematic diagram showing the state of bubbles released from the supply unit 31 of the example, and Fig. 9 is a schematic diagram showing the state of bubbles released from the supply unit 30 of the comparative example. Figs. 8 and 9 are each a partial cross-sectional view, and specifically, each show a side view of the supply unit while showing a cross-sectional view of the reservoir.

As a result, the water stored in the reservoir 50 in advance was introduced into the first flow channel 101 of the fluid handling device 10, passed through the first flow channel 101 and the tubes (circulation flow channel 40), and was then introduced into the supply unit 31. At this time, the fluid in the supply unit 31 flowed out through the outlet port 313 and the slit 314 into the water layer 70 in the reservoir 50. At the same time, as shown in Fig. 8, bubbles in the supply unit 31 were released through the slit 314 in the side surface of the supply unit 31 into the atmosphere in the reservoir 50. Specifically, from a region of the slit 314 of the supply unit 31 that was exposed above the water layer 70 in the reservoir 50, bubbles were released directly into the atmosphere. Meanwhile, from a region of the slit 314 that was located in the water layer 70 of the reservoir 50, bubbles escaped into the water layer 70 stored in the reservoir 50, rose in the water layer 70, and were released into the atmosphere above the water layer 70. In this manner, water circulation was repeated, and bubbles in the supply unit 31 were prevented from entering the first flow channel 101 of the fluid handling device 10. On the other hand, in the comparative example in which the supply unit 30 without a slit was used, as shown in the schematic diagram of Fig. 9, bubbles in the supply unit 30 came out through the outlet port 303 at the leading end of the supply unit 30, as with the fluid, accumulated near the outlet port 303, and combined with bubbles further coming out later, thus forming a large air mass 80 and remaining near the outlet port 303.

### (1-2)

In the present example, the reservoir 20 shown in Fig. 7 was used as the reservoir, and the inside of the reservoir 20 was made a closed system by forming the oil layer 71. Note that, unless otherwise stated, the present example was the same as that described in (1-1) above.

First, the substrate flow channel of the circulation flow channel 40 was set under the pump. One end of one of the tubes was connected to one end of the substrate flow channel, and the other end of that tube was connected to the outlet port 103 of the first flow channel 101 of the fluid handling device 10 shown in Fig. 1. The same chip as that described in (1-1) above was used as the fluid handling device 10. One end of the other tube was connected to the inlet port 312 of the supply unit 31, and the other end of the other tube was connected to the other end of the substrate flow channel.

Next, the leading end of the connection portion 204 of the reservoir 20 was connected to the inlet port 102 of the first flow channel 101 of the fluid handling device 10. Then, water containing a blue pigment was put into the reservoir 20 using a pipette until the position P(f) was reached, and 0.1 mL of mineral oil was put on top of the water using a pipette to form an oil layer 71 on the water layer 70. This oil layer 71 made the water layer 70 in the reservoir 20 a closed system. Next, the leading end of the supply unit 31 was disposed in the reservoir 20 so as to be located in the water layer 70, and, as in (1-1) above, the rotary pump was driven to circulate the water.

As a comparative example, as in (1-1) above, water was circulated in a similar manner using, instead of the supply unit 31 of the example above, the supply unit 30 shown in Fig. 16, which had the same size as the supply unit 31 and did not have the slit 314.

As a result, in the present example, as in the example described in (1-1) above, the water stored in the reservoir 20 in advance was introduced into the first flow channel 101 of the fluid handling device 10, passed through the first flow channel 101 and the circulation flow channel 40, and was then introduced into the supply unit 31. At this time, the fluid in the supply unit 31 flowed out through the outlet port 313 and the slit 314 into the water layer 70 in the reservoir 20. At the same time, bubbles in the supply unit 31 escaped through the slit 314 in the side surface of the supply unit 31 into the water layer 70 in the reservoir 20, rose in the water layer 70, passed through the oil layer 71 as well, and were released into the atmosphere. In this manner, water circulation was repeated, and bubbles in the supply unit 31 were prevented from entering the first flow channel 101 of the fluid handling device 10. On the other hand, in the comparative example in which the supply unit 30 without the slit 314 was used, as in the comparative example described in (1-1) above, bubbles in the supply unit 30 came out through the outlet port 303 at the leading end of the supply unit 30, as with the fluid, accumulated near the outlet port 303, and combined with bubbles further coming out later, thus forming a large air layer and remaining near the outlet port 303.

With respect to (1-1) and (1-2) above, the width W(s) of the slit of the supply unit 31 of the examples was changed to 0.5 mm, 1 mm, and 1.5 mm, and in these cases as well, similar results were obtained.

### [Example 2]

In the present example, the supply unit 32 having the rectangular vent portion 324 in Fig. 4A and the supply unit 33 having the elliptical vent portion 334 in Fig. 4B were used. With a fluid handling system that was the same as the fluid handling system of Example 1 (1-1) above unless otherwise stated, a fluid was introduced into the first flow channel of the fluid handling device via the reservoir with the lid portion, and the behavior of bubbles in the supply unit was observed.

The size of each supply unit was as follows. Note that, unless otherwise stated, the sizes of the various portions were the same as those of Example 1 above.
(1) Supply unit 32 in Fig. 4A
   Total length H(n): 20 mm
   Length from upper end P(s1) of vent portion 324 to outlet port 323: 12 mm
   Length from lower end of vent portion 324 to outlet port 323: 7 mm
   Length H(s) of vent portion 324: 5 mm
   Width W(s) of vent portion 324: 1.5 mm
   Inner diameter D(n): 1 mm
   Distance H(d1) between leading end of supply unit 32 and bottom surface 201 of reservoir 50: 6 mm
   Distance H(d2) between lower end of vent portion 324 and bottom surface 201 of reservoir 50: 10 mm
(2) Supply unit 33 in Fig. 4B
   Total length H(n): 20 mm
   Length from upper end P(s1) of vent portion 334 to outlet port 333: 12 mm
   Length from lower end of vent portion 334 to outlet port 333: 9 mm
   Major axis D(s1) of vent portion 334: 3 mm
   Minor axis D(s2) of vent portion 334: 1.5 mm
   Inner diameter D(n): 1 mm
   Distance H(d1) between leading end of supply unit 33 and bottom surface 201 of reservoir 50: 6 mm
   Distance H(d2) between lower end of vent portion 334 and bottom surface 201 of reservoir 50: 12 mm
(3) Reservoir 50
   Total volume: 1.5 mL
   Volume of main body 203: 1.4 mL
   Volume of connection portion 204: 0.06 mL
   Height H(r1) + H(r2) of main body 203: 20 mm
   Height H(r1) from bottom surface 201 to designed fluid level position P(f): 13 mm
   Height H(r2) from designed fluid level position P(f) to top opening: 7 mm.
   Inner diameter D(r1) of reservoir 50: 9 mm
   Diameter D(r2) of flow channel communicating portion 202 in bottom surface 201: 2 mm
   Height H(r3) of connection portion 204: 10 mm

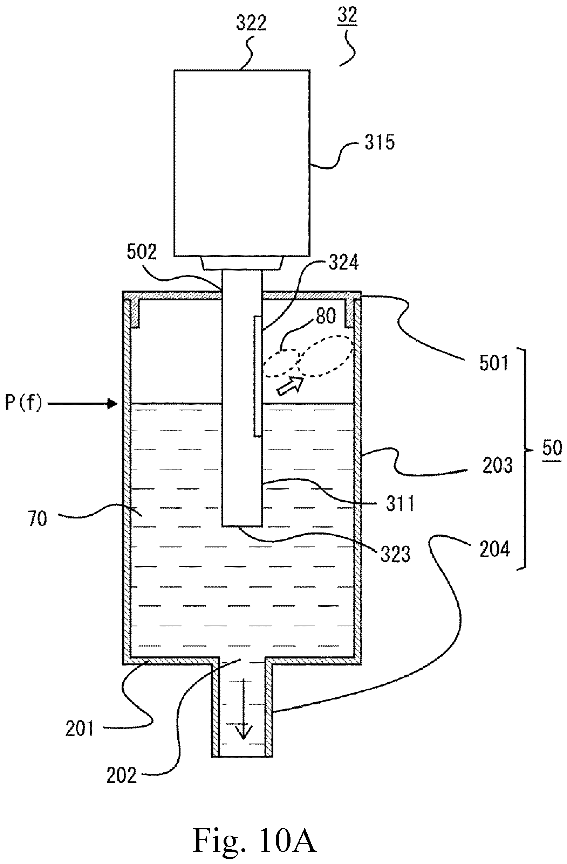

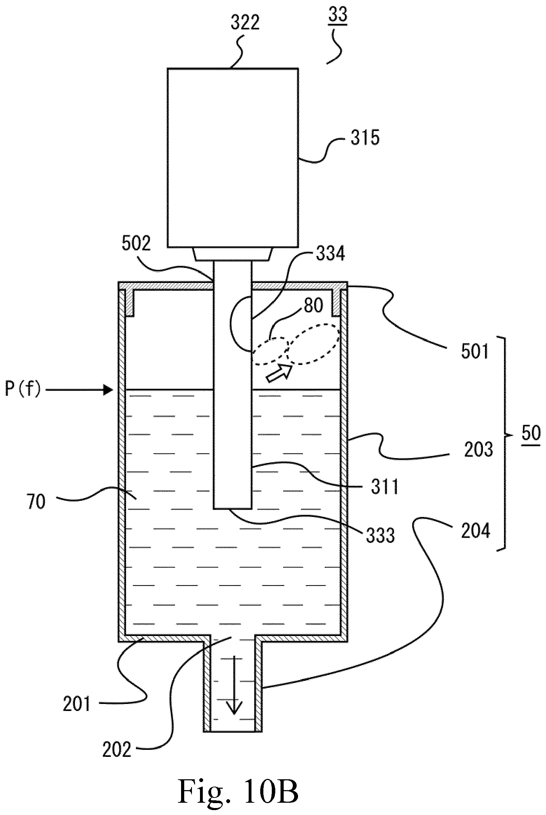

The results will be described using Figs. 10A and 10B. Fig. 10 is a schematic diagram showing the state of bubbles released from the supply unit 32 having the vent portion 324, which was the rectangular through hole in Fig. 4A, and Fig. 10B is a schematic diagram showing the state of bubbles released from the supply unit 33 having the vent portion 334, which was the elliptical through hole in Fig. 4B. Figs. 10A and 10B are each a partial cross-sectional view, and specifically, each show a side view of the supply unit while showing a cross-sectional view of the reservoir.

As a result, in the case where the supply unit 32 having the vent portion 324, which was the rectangular through hole, was used, water introduced into the supply unit 32 was caused to flow out through the vent portion 324 to the outside of the supply unit 32, gradually stored in the reservoir 50, and furthermore, caused to flow out from the reservoir 50 into the first flow channel 101 of the fluid handling device 10. At this time, as shown in Fig. 10A, bubbles in the supply unit 32 were released through the vent portion 324 in the side surface of the supply unit 32 into the atmosphere in the reservoir 50. Specifically, from a region of the vent portion 324 that was exposed above the water layer 70 in the reservoir 50, bubbles were released directly into the atmosphere. Meanwhile, from a region of the vent portion 324 that was located in the water layer 70 in the reservoir 50, bubbles escaped into the water layer 70, rose in the water layer 70, and were released into the atmosphere above the stored water layer 70.

The same was true for the case in which the supply unit 33 having the vent portion 334, which was the elliptical through hole, was used, and as shown in Fig. 10B, bubbles in the supply unit 33 were released through the vent portion 334 in the side surface of the supply unit 33 into the atmosphere in the reservoir 50. Specifically, from a region of the vent portion 334 that was exposed above the water layer 70 in the reservoir 50, bubbles were released directly into the atmosphere. Meanwhile, from a region of the vent portion 334 that was located in the water layer 70 in the reservoir 50, bubbles escaped into the water layer 70, rose in the water layer 70, and were released into the atmosphere above the stored water layer 70.

Although the present invention has been described above with reference to the embodiments, the present invention is not limited to the foregoing embodiments. Various changes that can be understood by those skilled in the art may be made to the configuration and detail of the present invention within the scope of the present invention.

As described above, in the fluid handling system of the present invention, since the supply unit has the vent portion, even when bubbles enter the supply unit along with the fluid, the bubbles are preferentially released through the vent portion to the outside, rather than through the outlet port, through which the fluid flows out. Therefore, when the fluid is caused to flow out of the supply unit, bubbles can be prevented from accumulating near the outlet port, through which the fluid flows out. Consequently, for example, the entry of bubbles into the flow channel of the fluid handling device as described above can be prevented.

### LIST OF REFERENCE NUMERALS

1, 2, 3: Fluid Handling System
10: Fluid Handling Device
101: First Flow Channel
102, 212, 302, 312, 322: Inlet Port
103, 213, 303, 313, 323: Outlet Port
14: Connection Portion
15: Valve
20, 21, 50: Reservoir
201, 211: Bottom Portion
202, 213: Communicating Portion (First Flow Channel Communicating Portion)
212: Communicating Portion (Circulation Flow Channel Communicating Portion)
203: Main Body
204: Connection Portion
30, 31, 32, 33, 34: Supply Unit
314: Slit (Vent Portion)
315: Holding Portion
324, 334: Through Hole (Vent Portion)
342: Opening (Inlet Port)
346: Opening (Vent Portion)
345: Slit (Outlet Port)
40, 41: Circulation Flow Channel
401, 402: Branch Flow Channel
413: Downstream Region
414: Contact Region
416: End Portion
51: Fluid Chamber
52: Collection Chamber
501: Lid Portion
60: Main Body
601, 603: Cover
602: Substrate
70: Fluid Layer
71: Oil Layer
80: Bubble

## Claims

1. A fluid handling system comprising a fluid handling device, a reservoir that stores a fluid, and a supply unit that supplies the fluid to the reservoir,
wherein the fluid handling device has a first flow channel,
the reservoir has a main body,
the main body having a communicating portion that communicates with the first flow channel of the fluid handling device, and
the supply unit has a second flow channel through which the fluid flows and a vent portion that makes the second flow channel communicate with an outside,
the second flow channel having an inlet port through which the fluid flows into the second flow channel and an outlet port through which the fluid flows out of the second flow channel,
the vent portion being located above the outlet port,
the outlet port being located inside the reservoir, and
the vent portion being located above the communicating portion.

2. The fluid handling system according to claim 1, wherein the vent portion is a slit or a through hole.

3. The fluid handling system according to claim 2, wherein an end portion of the vent portion on the inlet port side is located above a fluid level position to which the fluid is designed to be stored in the reservoir.

4. The fluid handling system according to any one of claims 1 to 3,
wherein a ratio of a cross-sectional area (R) of the main body of the reservoir to a cross-sectional area (T) of the supply unit is 1.5 or more, and
the cross-sectional area (T) and the cross-sectional area (R) are each the area of a horizontal cross section passing through an end portion of the vent portion on the inlet port side.

5. The fluid handling system according to any one of claims 1 to 4, wherein the main body of the reservoir is open at its top.

6. The fluid handling system according to any one of claims 1 to 5, further comprising a circulation flow channel,
wherein the fluid handling device has an inlet port through which the fluid from the reservoir flows into the fluid handling device and an outlet port through which the fluid flows out of the fluid handling device, and
the outlet port of the fluid handling device and the inlet port of the supply tube are connected to each other by the circulation flow channel.

7. The fluid handling system according to any one of claims 1 to 6, further comprising a fluid delivery unit.

8. A supply unit for a fluid handling system, which is a fluid supply unit for the fluid handling system according to any one of claims 1 to 7, the supply unit comprising a flow channel through which a fluid flows and a vent portion that makes the flow channel communicate with an outside,
wherein the flow channel has an inlet port through which the fluid flows into the flow channel and an outlet port through which the fluid flows out of the flow channel, and
the vent portion is located above the outlet port.

9. The supply unit for the fluid handling system according to claim 8, wherein the vent portion is a slit or a through hole.

10. A kit for the fluid handling system according to any one of claims 1 to 7, the kit comprising a reservoir that stores a fluid and a supply unit that supplies the fluid to the reservoir,
wherein the reservoir has a main body,
the main body having a communicating portion that communicates with the first flow channel of the fluid handling device, and
the supply unit has a flow channel through which the fluid flows and a vent portion that makes the flow channel communicate with an outside,
the flow channel having an inlet port through which the fluid flows into the flow channel and an outlet port through which the fluid flows out of the flow channel,
the vent portion being located above the outlet port,
the outlet port being located inside the reservoir, and
the vent portion being located above the communicating portion.
